# EUROPEAN PATENT APPLICATION

(11) **EP 1 839 656 A1**
(43) Date of publication of application: **03.10.2007**
(21) Application number: 06006847.5
(22) Date of filing: 31.03.2006
(51) Int. Cl.: A61K 31/19, A61P 17/10

(54) **Use of valproic acid for the topical treatment of mild to moderate acne vulgaris**

(71) Applicant: TopoTarget Germany AG, 60596 Frankfurt am Main (DE)
(72) Inventor: Schwarz, Sylvia E., 60322 Frankfurt am Main (DE); Hentsch, Bernd, 60322 Frankfurt am Main (DE)
(74) Representative: Kalhammer, Georg

(57) **Abstract**

The present invention provides a surprising therapeutic beneficial use for the topical application of valproic acid as a single agent therapy for patients suffering from mild to moderate acne vulgaris. Topically applied VPA has a clinical efficacy comparable to that of the marketed standard medication for this indication, isotretinoin. Furthermore, topically applied VPA is on average well to very well tolerated. The invention relates to the topical medical use of VPA for the treatment of acne vulgaris and comprises the topical application of VPA or of a pharmaceutically acceptable salt thereof.

## Description

The present invention provides a surprising therapeutic beneficial use for the topical application of valproic acid as a single agent therapy for patients suffering from mild to moderate acne vulgaris. Therapeutic beneficial means that VPA topically applied has a comparable clinical efficacy as the marketed standard medication for this indication, isotretinoin. The invention relates to the topical medical use of VPA for the treatment of acne vulgaris and comprises the topical application of VPA or of a pharmaceutically acceptable salt thereof, and a dermatologically acceptable carrier. Furthermore, topically applied VPA is on average well to very well tolerated.

### BACKGROUND OF THE INVENTION

### Chromatin Regulation and Disease

Local remodeling of chromatin is a key step in the transcriptional activation of genes. Dynamic changes in the nucleosomal packaging of DNA must occur to allow transcriptional proteins to make contact with the DNA template. One of the most important mechanisms influencing chromatin remodeling and gene transcription are posttranslational modifications of histones and other cellular proteins by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). In the case of histone hyperacetylation, changes in the electrostatic attraction of DNA and steric hindrance introduced by the hydrophobic acetyl group lead to destabilisation of the interaction of histones with DNA. As a result, acetylation of histones disrupts nucleosomes and allows the DNA to become accessible to the transcriptional machinery. Removal of the acetyl groups allows the histones to bind more tightly to DNA and to adjacent nucleosomes, and thus, to maintain a transcriptionally repressed chromatin structure. Acetylation is mediated by a series of enzymes with histone acetyltransferase (HAT) activity. Conversely, acetyl groups are removed by specific histone deacetylase (HDAC) enzymes. Disruption of these mechanisms gives rise to transcriptionally misguided regulation and may contribute to a variety of human diseases, including autoimmune, inflammatory, metabolic or hyperproliferative disorders, including tumorigenic transformation and tumor progression.

### Nuclear Receptors and Histone Deacetylases

Nuclear hormone receptors are ligand-dependent transcription factors that control development and homeostasis through both positive and negative control of gene expression. Defects in these regulatory processes underlie the causes of many diseases and play an important role in the development of cancer. Many nuclear receptors, including T3R, RAR and PPAR, can interact with corepressors, such as N-CoR and SMRT, in the absence of a ligand and thereby inhibit transcription. Furthermore, N-CoR has also been reported to interact with antagonist-occupied progesterone and estrogen receptors. Most interestingly, N-CoR and SMRT have been shown to exist in large protein complexes, which also contain mSin3 proteins and histone deacetylases (Pazin and Kadonaga, 1997; Cell 89, 325-8). Thus, the ligand-induced switch of nuclear receptors from repression to activation reflects the exchange of corepressor and coactivator complexes with antagonistic enzymatic activities.

### The Protein Family of Histone Deacetylases

The recruitment of histone acetyltranferases (HATs) and histone deacetylases (HDACs) is considered as a key element in the dynamic regulation of many genes playing important roles in cellular proliferation and differentiation. Hyperacetylation of the N-terminal tails of histones H3 and H4 correlates with gene activation whereas deacetylation can mediate transcriptional repression. Consequently, many diseases have been linked to changes in gene expression caused by mutations affecting transcription factors. Aberrant repression by leukemia fusion proteins such as PML-RAR, PLZF-RAR, AML-ETO, and Stat5-RAR serves as a prototypical example in this regard. In all of these cases, chromosomal translocations convert transcriptional activators into repressors, which constitutively repress target genes important, e.g., for hematopoietic differentiation via recruitment of HDACs. It is plausible that similar events could also contribute to pathogenesis in many other types of diseases. There is growing evidence that the same holds true also for autoimmune, inflammatory, metabolic or hyperproliferative disorders.

Mammalian histone deacetylases can be divided into three subclasses (Gray and Ekström, 2001). HDACs 1, 2, 3, and 8 which are homologues of the yeast RPD3 protein constitute class I. HDACs 4, 5, 6, 7, 9, and 10 are related to the yeast Hda 1 protein and form class II. Recently, several mammalian homologues of the yeast Sir2 protein have been identified forming a third class of deacetylases which are NAD dependent. Furthermore, HDAC11 has been classified as a class I histone deacetylase with structural features of a class II HDAC. All of these HDACs appear to exist in the cell as subunits of a plethora of multiprotein complexes. In particular, class I and II HDACs have been shown to interact with transcriptional corepressors mSin3, N-CoR and SMRT which serve as bridging factors required for the recruitment of HDACs to transcription factors.

### Therapy with HDAC Inhibitors

Additional clinical investigations have recently been initiated to exploit the systemic clinical treatment of cancer patients based on the principle of HDAC (histone deacetylase) inhibition. By now, a clinical phase II trial with the closely related butyric acid derivative Pivanex (Titan Pharmaceuticals) as a monotherapy has been completed demonstrating activity in stage III/IV non-small cell lung cancer (Keer et al., 2002, ASCO, Abstract No. 1253). More HDAC inhibitors have been identified, with NVP-LAQ824 (Novartis), SAHA (Aton Pharma Inc., now Merck & Co.) and PXD101 (TopoTarget A/S) being members of the structural class of hydroxamic acids tested in phase II clinical trials (Marks et al., 2001, Nature Reviews Cancer 1, 194-202). Another class comprises cyclic tetrapeptides, such as depsipeptide (FR901228 - Fujisawa) used successfully in a phase II trial for the treatment of T-cell lymphomas (Piekarz et al., 2001, Blood 98, 2865-8). Furthermore, MS-27-275 (Mitsui Pharmaceuticals), a compound related to the class of benzamides, is now being tested in a phase I trial treating patients with hematological malignancies.

### Valproic acid as an inhibitor of histone deacetylases

Valproic acid (VPA) has been developed as a drug used for the treatment of epilepsia. Accordingly, VPA is used systemically, orally, or intravenously, to allow the drug to pass the blood brain barrier to reach the epileptic target regions in the brain tissue in order to fulfill its anti-epileptic mission. Moreover, VPA has been shown to potentially possess particular beneficial effects when used for the treatment of many different types of human cancers as a single agent or in combination with a whole variety of other anti-tumor therapies which are individually based on strikingly different modes of action, by inhibiting specific sets of enzymes having HDAC activity and thereby inducing differentiation and/or apoptosis (WO 02/07722 A2, EP 1170008; WO 03/024442 A2, EP 1293205 A1). For the treatment or prevention of malignant diseases, autoimmune diseases, or other inflammatory or hyperproliferative disorders, VPA may also be administered systemically, orally, or intravenously. Furthermore, it was shown, that VPA permeates human skin effectively and therefore can be administered topically on skin exhibiting beneficial effects when used for the topical treatment or prevention of autoimmune, inflammatory or hyperproliferative human skin diseases, e.g., psoriasis and human skin cancer (WO 2005/000289).

### Valproic Acid

Valproic acid (VPA; 2-propyl-pentanoic acid = 2PPA) has multiple biological activities which depend on different molecular mechanisms of action:
- VPA is an antiepileptic drug.
- VPA is teratogenic. When used as an antiepileptic drug during pregnancy, VPA can induce birth defects (neural tube closure defects and other malformations) in a few percent of born children. In mice, VPA is teratogenic in the majority of mouse embryos when properly dosed.
- VPA activates a nuclear hormone receptor (PPARδ). Several additional transcription factors are also derepressed but some factors are not significantly derepressed (glucocorticoid receptor, PPARα).
- VPA occasionally causes hepatotoxicity, which may depend on poorly metabolized esters with coenzyme A.
- VPA is an inhibitor of HDACs.
- VPA modulates the cytokine expression profile of cells, e.g. immune cells.
- VPA can suppress the secretion of inflammatory cytokines of cells, e.g. immune cells.

The use of VPA derivatives allowed to determine that the different activities are mediated by different molecular mechanisms of action. Teratogenicity and antiepileptic activity follow different modes of action because compounds could be isolated which are either preferentially teratogenic or preferentially antiepileptic (Nau et al., 1991, Pharmacol. Toxicol. 69, 310-321). Activation of PPARδ was found to be strictly correlated with teratogenicity (Lampen et al., 1999, Toxicol. Appl. Pharmacol. 160, 238-249) suggesting that, both, PPARδ activation and teratogenicity require the same molecular activity of VPA. Also, differentiation of F9 cells strictly correlated with PPARδ activation and teratogenicity as suggested by Lampen et al., 1999, and documented by the analysis of differentiation markers (Werling et al., 2001, Mol. Pharmacol. 59, 1269-1276). It was shown, that PPARδ activation is caused by the HDAC inhibitory activity of VPA and its derivatives (PCT/EP01/07704; WO 03/024442 A2). Furthermore, it was shown that the established HDAC inhibitor TSA activates PPARδ and induces the same type of F9 cell differentiation as VPA. From these results it can be concluded that not only activation of PPARδ but also induction of F9 cell differentiation and teratogenicity of VPA or VPA derivatives are caused by HDAC inhibition.

Antiepileptic and sedating activities follow different structure activity relationships and thus obviously depend on a primary VPA activity distinct from HDAC inhibition. The mechanism of hepatotoxicity is poorly understood, and it is unknown whether it is associated with formation of the VPA-CoA ester. HDAC inhibition, however, appears not to require CoA ester formation.

### Retinoids

Retinoic Acid (RA) and derivatives belong to a class of compounds known as retinoids, including but not limited to 9-cis RA, isotretinoin, trans RA, all-trans RA, Tazaratone (Guenther, 2003, Am. J. Clin. Dermatol. 4, 197-202; Peris K et al., 1999, N Engl J Med 341, 1767-1768), that play key roles in regulating gene transcription and, therefore, govern multiple functions in the body, such as cell division and differentiation, immune response and embryonic development. They also control the development and spread of cancer cells, and some, including RA, can inhibit tumor growth by preventing cancer cell proliferation (Altucci and Gronemeyer, 2001, Nature Reviews Cancer 1, 181-193).

Specific retinoids are also approved for the medical therapy of a number of diseases which are based at least in part on misregulated immune cell functions. Such indications include, e.g., acne vulgaris and psoriasis.

The effects of retinoids are mainly mediated by two classes of nuclear receptors, the RA receptors (RARs) and retinoid X receptors (RXRs) (Zhang & Pfahl 1993, Kastner *et al.* 1995, Mangelsdorf & Evans 1995). RARs and RXRs are encoded by three distinct genes (α, β and γ). In addition, many retinoid receptor isoforms are generated through differential promoter usage, giving rise to a large number of distinct retinoid receptor proteins. To date, there are dozens of receptors which are known to mediate the effect of retinoids. 9-cis RA is a high-affinity ligand for both RARs and RXRs, whereas *trans-RA* is a ligand for only RARs. Retinoid receptors belong to a large steroid/thyroid receptor superfamily that mediate the biological effects of many hormones, vitamins and drugs. RARs and RXRs act as transcriptional factors to positively or negatively regulate expression of target genes by binding to their response elements (RAREs) located in promoter regions of the target genes of an active chromatin structure and results in the transcription of target genes. In addition to their direct effects on transcription, liganded RAR can modulate the activity of other transcriptional factors, such as AP-1 (Pfahl 1993). Activated retinoid receptors can inhibit the activity of AP-1, thereby regulating the expression of AP-1 target genes. The inhibition of AP-1 activity is linked to the anti-proliferative effects of retinoids, and appears to be separable from their direct activation of transcription of retinoid-target genes.

For some time doctors have been using RA derivatives, to treat several cancers, particularly prostate cancer and leukemia, and they are now experimenting with the drug to also treat other types of cancer. A typical treatment with RA seeks to activate RAR in order to switch on favorable genes. The great drawback to RA, however; is that it requires high levels of the medication in order to turn genes 'on' and 'off,' often triggering devastating and potentially fatal side effects (Altucci and Gronemeyer, 2001, Nature Reviews Cancer 1, 181-193).

RA or other retinoids such as Tazarotene (Guenther, 2003, Am. J. Clin. Dermatol. 4, 197-202) may also be used topically for the treatment of mild to moderate acne and sun damage (photoaged) skin. Retinoids have even been used to treat certain skin cancers, such as Basal Cell Carcinoma (Peris K et al., 1999, N Engl J Med 341, 1767-1768). After topical administration, RA appears to increase the rate of cell division and turnover. The number of cell layers in the outer portion of the skin is decreased. When pimples are present, they are more rapidly resolved. Topical RA is effective in reducing fine wrinkling, mottled hyperpigmentation, and roughness associated with overexposure to the sun. The results of treatment are not immediate and may be visible only after weeks to months. Discontinuation of treatment usually results in a loss of RA effects.

### The role of cytokines in autoimmunity and inflammation

Cytokines are a diverse group of soluble proteins and peptides which act as regulators to modulate the functional activities of individual cells and tissues. They are designed to induce an inflammatory reaction as a defense against foreign or altered endogenous substances. In many respects the biological activities of cytokines resemble those of classical hormones produced in specialized glandular tissues by acting at a systemic level to induce biological phenomena such as inflammation, acute phase reaction and autoimmunity. However, inappropriate activation of inflammatory responses is the underlying cause of many common diseases and inflammatory reactions are, therefore, also an important target for drug development.

A number of cytokines accelerate inflammation and regulate a local or systemic inflammatory reaction either directly or through their ability to induce the synthesis of cellular adhesion molecules or other cytokines in certain cell types. The major cytokines that are responsible for early responses are IL1, IL6 and TNF-alpha. Other pro-inflammatory mediators include LIF, IFN-gamma, GM-CSF, IL11, IL12, IL18, and a variety of other chemokines.

However, the role of cytokines is not restricted to the inflammatory process alone, but have also a leading role in the development and propagation of autoimmune diseases. A classical example is rheumatoid arthritis where specific CD4+ T cells, most likely as a response to an unknown exogenous or endogenous antigen, induce an immune response in affected joints (Olsen et al., 2003, New England Journal of Medicine 350, 2167-79). Consequently, recruited monocytes, macrophages, and fibroblasts produce cytokines such as tumor necrosis factor-α (TNF-α) and interleukin-1 within the synovial cavity. These cytokines are central to a damaging cascade, ultimately triggering the production of matrix metalloproteinases and osteoclasts, which results in irreversible damage to soft tissues and bones.

TNF-α, an inflammatory cytokine that is released by activated monocytes, macrophages, and T lymphocytes, promotes inflammatory responses that are important in the pathogenesis of rheumatoid arthritis. Patients with rheumatoid arthritis have high concentrations of TNF-α in the synovial fluid. TNF-α is localized to the junction of the inflammatory pannus and healthy cartilage, and high synovial fluid TNF-α concentrations are associated with the erosion of bone.

Not surprisingly, TNF antagonists appear to be among the most effective treatments available for rheumatoid arthritis. The response is generally rapid, often occurring within a few weeks, although not all patients have a response.

Agents directed against TNF-α are not only effective in the treatment of chronic autoimmune disorders such as rheumatoid arthritis, but also in the treatment of Chrohn's disease, ulcerative colitis, Sjögren's syndrome, scleroderma, psoriatic arthritis, ankylosing spondylitis, refractory uveitis, Behçet's disease, adult-onset Still's disease, and Wegener's granulomatosis.

Another example is psoriasis, where a T cell mediated immune response is directed against keratinocytes. These T lymphocytes encounter the initiating antigen in the dermis or epidermis, and secrete type-1 cytokines (Th1), particularly interferon-γ, interleukin 2, and TNF-α. These secretions result in proliferation and decreased maturation of keratinocytes and associated vascular changes. Secretion of other cytokines such as interleukin 8 contribute to the complete picture of psoriasis (Lebwohl, 2004, The Lancet 361, 1197-1204).

Further evidence for the causal involvement of cytokines in autoimmune diseases has come from observations made after the use of cytokines in the treatment of various diseases (Krause et al., 2003, The American Journal of Medicine 115, 390-397). Interestingly, they are associated with side effects such as the triggering and exacerbation of immune and autoimmune conditions, which may evolve into overt autoimmune disorders. These autoimmune manifestations seem to be more common in patients with a pre-existing tendency towards autoimmunity.

Exacerbation of multiple sclerosis has been observed during treatment with interferon-γ. The frequency of autoimmune manifestations associated with interferon-γ therapy seems to be low but there have been reports of systemic lupus erythematosus in patients treated with interferon-γ alone, as well as in combination with interferon-γ for myeloproliferative disorders. Interferon-γ is involved in the pathogenesis of systemic lupus erythematosus in animal models. Administration of interferon-γ accelerates the rate of progression to glomerulonephritis in lupus-prone (NZBXNZvV)F1 mice, which is prevented by treatment with anti-interferon-γ antibodies. Elevated serum levels of interferon-γ have been reported in patients with systemic lupus erythematosus. Interferon-γ is produced by natural killer cells and binds to the type II interferon receptor. It is less effective than interferon-γ in activating natural killer cells and has less potent antiviral and antitumor effects. However, interferon-γ is the most potent inducer of macrophage activation and major histocompatibility class II molecules. It stimulates immunoglobulin secretion by B cells and promotes T-cell differentiation towards the T helper 1 type.

Interleukin 2 is secreted by activated T cells with antitumor activity. It is effective in the treatment of metastatic malignant melanoma and renal cell carcinoma. It induces T-cell proliferation, potentiates B-cell growth, and enhances natural killer cell and monocyte activation. The most common autoimmune side effect seen under interleukin 2 therapy is immune-mediated thyroid disease. Reversible thyroid dysfunction occurs frequently in patients with cancer who are treated with interleukin either alone or in conjunction with lymphokine-activated killer cells or interferon-γ. In a study with interleukin 2 in patients with metastatic renal cell carcinoma, antithyroid antibodies were detected in 18% (60/329) of patients. Other much less common phenomena that may be considered autoimmune have been described in association with interleukin 2 therapy. These include rheumatoid arthritis, psoriatic arthropathy, ankylosing spondylitis, and Reiter's syndrome. The triggering of arthritis may be explained by induction of autoantigen recognition by T cells infiltrating the joints, leading to inflammation. Interleukin 2 may potentiate a breakdown of immunologic tolerance to muscle-specific and tumor antigens, resulting in destruction of both tumor and muscle cells. One patient with metastatic renal cell carcinoma treated with interleukin 2 and lymphokine-activated killer cells developed an acute exacerbation of systemic sclerosis. Serum levels of interleukin 2 and soluble interleukin 2 receptor are elevated in patients with systemic sclerosis and correlate with disease duration and activity. These observations may explain the association between interleukin 2 therapy and the development of systemic sclerosis.

### VPA and acne vulgaris

A huge body of clinical experience has accumulated over almost three decades of use of VPA for the treatment and prevention of epileptic seizures. The drug is approved for oral and intravenous administration. Systemic application of VPA is considered to be safe and well tolerated even in long term treatment in a dose range of approx. 1200 mg - 2100 mg/day leading to serum concentrations between 0.3 to 0.9 mM. The most commonly reported adverse effects are gastrointestinal disturbances including diarrhoea, nausea and vomiting. Neurological effects observed include sedation, ataxia and tremor. These symptoms occur infrequently and usually respond to a decrease in dosage. Rash, acne, alopecia and stimulation of appetite have been observed occasionally. In very rare cases, fatal hepatic failure and pancreatitis have also been reported, usually seen in patients with pre-existing hepatic or pancreatic dysfunction.

Acne is a well documented resultant side effect seen in patients taking VPA as an anti-epileptic agent (Genton et al, 2001 Epilepsia 42:295-304; Morrell 2003, Epilepsia 44 [Suppl. 4] 11-20). Acne is a common skin disease that affects 85-100% of people at some time during their lives, with a high prevalence in the population of 15 to 24 years of age. Acne lesions usually occur on the face, neck, back, chest, and shoulders.

Follicular epidermal hyperproliferation and hyperkeratinization appear to be one of the primary events in the development of an acne lesion. This hyperproliferation may be stimulated by an alteration in sebum and lipid levels in acne lesions and leads to plugged pores (blackheads and whiteheads), inflamed pimples (pustules), and deeper lumps (cysts or nodules).

The present invention aims at providing an improved treatment for the prevention or treatment of acne.

VPA has been developed as a drug used for the treatment of epilepsia. Accordingly, VPA is used systemically, orally or intravenously, to allow the drug to pass the blood brain barrier to reach the epileptic target regions in the brain tissue in order to fulfill its anti-epileptic mission. Therefore, VPA is regarded as a drug that must be applied systemically in order to achieve its therapeutic effects.

Surprisingly, it was now found that VPA has in fact unexpected beneficial effects when used for the topical treatment of acne. Here, the precise mode of action which is employed by VPA is not fully understood, but VPA has potential to inhibit the release of cytokines in the inflammatory response. This surprising potential of VPA may be based on its activity as an inhibitor of specific sets of enzymes having HDAC activity.

This finding is very surprising in view of the fact that acne is a well known side effect seen in patients taking VPA as an epileptic (see supra). Furthermore, there are no previous reports on the use of VPA or any other HDAC inhibitor for the in vivo therapy of acne.

### SUMMARY OF THE INVENTION

The present invention provides a surprising new medical use for patients suffering from acne vulgaris or other types of acne when VPA is applied topically as the single therapeutic agent.

The invention therefore relates to the use of a histone deacetylase inhibitor for the manufacture of a medicament for the topcial treatment of acne.

The medicament can be used for the treatment of non-inflammatory and inflammatory acne lesions.

A preferred histone deacetylase inhibitor is valproic acid (VPA), or pharmaceutically acceptable derivatives or prodrugs thereof. Further histone deacetylase inhibitors include hydroxamic acid derivatives, benzamides, pyroxamides and derivatives thereof, microbial metabolites exhibiting HDAC inhibitory activity, fatty acids and derivatives thereof, cyclic tetrapeptides, peptidic compounds, HDAC class III inhibitors and SIRT inhibitors. Examples are LAQ824, LBH589, Trichostatin A, Suberoyl anilide hydroxamic acid, CBHA, Pyroxamide, Scriptaid, CI-994, CG-1521, Chlamydocin, Biaryl hydroxamate such as A-161906, Bicyclic aryl-N-hydroxycarboxamides, PXD-101, MGCD0103, TPX-HA analogue (CHAP), Oxamflatin, Trapoxin, Depudecin, Apidicin, MS-275, Pivanex (Pivaloyloxymethyl butyrate), trapoxin A, Depsipeptide (FK-228), Tacedinaline, MG2856.

Preferably, the composition which is topically applied to the skin of the patient comprises the histone deacetylase inhibitor (e.g. VPA) at a concentration of from 1% to 4% by weight, and the active ingredient is suspended in a pharmaceutically acceptable carrier.

In a preferred aspect, the histone deacetylase inhibitor is topically applied once to three times daily at an accumulated total daily dose of from 0.5 mg to 200 mg per 1 cm² of lesion, more preferably of from 2.0 mg to 50 mg per 1 cm² of lesion. The total amount of topically applied medicament may be 2.000 mg or less per day, and the total amount of the topically applied histone deacetylase inhibitor may be 60 mg or less per day.

The serum concentration of the histone deacetylase inhibitor in a treated patient is usually less than 12.0 µg/ml, preferably less than 4 µg/ml, after repeated topical application over a period of at least 56 days, e.g. over a period of from 56 to 85 days.

Surprisingly, the treatment shows very good tolerability, e.g. it does usually not cause erythema or pain at the site of application. Generally, the local tolerability of the medicament is higher than that of isotretinoin.

The treatment may further comprise administration of a further active agent in a combination therapy. The further active agent may be a histone deacetylase inhibitor other than VPA or it may be a compound that is not an HDAC inhibitor. Examples of further active agents include antibiotics, retinoids, hormonal agents and topical bactericidals such as benzoyl peroxide.

In another embodiment, the histone deacetylase inhibitor (e.g. VPA) is the only active agent in said treatment, i.e. it is used in a monotherapy for treating acne.

The invention further relates to a pharmaceutical composition comprising (i) a first active agent which is a histone deacetylase inhibitor, and (ii) a second active agent which is selected from the group consisting of histone deacetylase inhibitors other than the first active agent, antibiotics, retinoids, hormonal agents and topical bactericidals. The invention further relates to a pharmaceutical kit for the treatment of acne, comprising (i) a first active agent which is a histone deacetylase inhibitor, and (ii) a second active agent which is selected from the group consisting of histone deacetylase inhibitors other than the first active agent, antibiotics, retinoids, hormonal agents and topical bactericidals.

In general, the present invention provides novel possibilities to treat acne vulgaris. Applicants found that the HDAC inhibitory and cellular differentiation-inducing activity of VPA can be used successfully alone, i.e. as a novel monotherapy treatment, for the topical treatment of acne vulgaris, thereby displaying good local tolerability for patients.

### DETAILED DESCRIPTION OF THE INVENTION AND OF EXEMPLARY EMBODIMENTS

The present invention relates to the use of a histone deacetylase (HDAC) inhibitor for the manufacture of a medicament for the treatment of acne. Preferably, the treatment is a topical treatment, i.e. it comprises the topical application of the medicament. The term "topical application", as used herein, means to apply or spread the compositions described herein onto the surface of the skin.

### Acne

There are various types of acne including acne vulgaris, acne rosacea, acne conglobata, acne fulminans, chemical acne, chloracne, tropical acne, acne inversa and corticosteroid acne. According to this invention, the HDAC inhibitor can be used to treat any of these types of acne. Preferably, the HDAC inhibitor is used to treat acne vulgaris.

Acne vulgaris is a chronic disease of sebaceous follicles that is multifactorial in etiology and varies in severity as evidenced by lesion type, size, numbers, scarring, and post-inflammatory pigmentary changes. Acne occurs more frequently on the face, but can also occur on nonfacial skin (e.g., back, shoulders, chest).

There are two major types of acne lesions: noninflammatory and inflammatory. Noninflammatory lesions of acne are the open (blackheads) or closed (whiteheads) comedones. These lesions, especially closed comedones, may be precursors to the larger inflammatory lesions and therefore are of clinical importance. Inflammatory lesions are divided into papules, pustules, and nodules/nodulocystic lesions, depending on the severity and location of the inflammation within the dermis. The papules and pustules have surrounding halos of erythema allowing for their characterization as inflammatory. Nodules are typically erythematous and often tender and/or painful. Additionally, they are deep-seated in the skin (i.e., centered in the dermis or subcutis). Nodules have been defined as being greater than 5 mm in diameter.

In one aspect, the HDAC inhibitor is used to treat inflammatory acne lesions. In a preferred embodiment, the HDAC inhibitor is used to treat non-inflammatory acne lesions. In a third embodiment, the HDAC inhibitor is used to treat inflammatory and non-inflammatory acne lesions.

Acne vulgaris can appear in various forms depending on the severity of the disease.
● Mild Acne - Superficial lesions, mostly comedones, and some inflamed papules and pustules, no evidence of scarring and no nodules.
● Moderate Acne - Lesions may involve the face and trunk, mild superficial scarring may be seen, with papules, pustules, comedones, and a few nodules. Nodules are not the dominant lesion.
● Severe Acne - Acne is causing persistent painful nodules, comedones, papules, and may have deep scars.
   (see: http://www.mamc.amedd.army.mil/Referral/guidelines/derm_acne.htm)

The HDAC inhibitor (e.g. VPA) can be used to treat mild, moderate and/or severe acne vulgaris. It is preferably used, however, in the treatment of mild or moderate acne vulgaris.

The patient to be treated can be of any age. Preferably, the patient has an age of from 13 to 40 years, preferably of from 14 to 30 years, most preferably of from 15 to 25 years.

### HDAC inhibitor

An HDAC inhibitor is a compound that is capable of inhibiting the enzymatic activity of one or more HDAC enzyme(s). The inhibitory acitivity of a histone deacetylase inhibitor can be determined in an in vitro assay as described in Example 1 of WO 2005/105055 the contents of which is incorporated herein by reference. The IC₅₀ value can be taken as a measure for the inhibitory activity of a histone deacetylase inhibitor. A low IC₅₀ value indicates a high inhibitory activity; a high IC₅₀ value indicates a low inhibitory activity. The histone deacetylase inhibitors used in accordance with this invention preferably have an IC₅₀ value of less than 1 mM, more preferably of less than 500 µM with respect to at least one histone deacetylase.

The preferred HDAC inhibitor used in accordance with this invention is VPA or a pharmaceutically acceptable derivative or prodrug therof. Derivatives of VPA in particular include pharmaceutically acceptable salts of VPA.The most preferred HDAC inhibitors are VPA and pharmaceutically acceptable salts thereof.

The invention therefore also concerns the use of VPA or a derivative thereof for the manufacture of a medicament for a topical treatment of diseases as listed above. The derivatives of VPA include carbon branched carboxylic acids or carboxylic acid derivatives as described by the following formula (1) 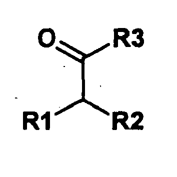 wherein R1 and R2 independently are a linear or branched, saturated or unsaturated aliphatic C₃₋₂₅ hydrocarbon chain which optionally comprises one or several heteroatoms and which may be substituted, R3 is hydroxyl, halogen, alkoxy or an optionally alkylated amino group. Different R1 and R2 residues give rise to chiral compounds. Usually one of the stereo isomers has a stronger teratogenic effect than the other one (Nau et al., 1991, Pharmacol. Toxicol. 69, 310-321) and the more teratogenic isomer more efficiently activates PPARδ (Lampen et al., 1999). Therefore, this isomer can be expected to inhibit HDACs more strongly (PCT/EP01/07704). The present invention encompasses the racemic mixtures of the respective compounds and in particular the more active isomers. The hydrocarbon chains R1 and R2 may comprise one or several heteroatoms (e.g. O, N, S) replacing carbon atoms in the hydrocarbon chain. This is due to the fact that structures very similar to that of carbon groups may be adopted by heteroatom groups when the heteroatoms have the same type of hybridization as a corresponding carbon group.

R1 and R2 may be substituted. Possible substituents include hydroxyl, amino, carboxylic and alkoxy groups as well as aryl and heterocyclic groups.

Preferably, R1 and R2 independently comprise 3 to 10, more preferably 4 to 10 or 5 to 10 carbon atoms. It is also preferred that R1 and R2 independently are saturated or comprise one double bond or one triple bond. In particular, one of the side chains (R1) may preferably contain sp1 hybridized carbon atoms in position 2 and 3 or heteroatoms which generate a similar structure. This side chain should comprise 3 carbon or heteroatoms but longer chains may also generate HDAC-inhibiting molecules. Also, inclusion of aromatic rings or heteroatoms in R2 is considered to generate compounds with HDAC inhibitory activity because the catalytic site of the HDAC protein apparently accommodates a wide variety of binding molecules. With the observation that teratogenic VPA derivatives are HDAC inhibitors, also compounds which have previously been disregarded as suitable antiepileptic agents are considered as HDAC inhibitors (PCT/EP01/07704). In particular, but not exclusively, compounds having a propinyl residue as R1 and residues of 7 or more carbons as R2, are considered (Lampen et al, 1999).

Preferably, R3 is OH and forms a carboxylic group together with the adjacent carbonyl group. Also derivatization of the carboxylic group has to be considered for generating compounds with potential HDAC inhibitory activity. Such derivatives may be halides (e.g. chlorides), esters or amides.

When R3 is alkoxy, the alkoxy group comprises 1 to 25, preferably 1 to 10 carbon atoms. When R3 is a mono- or di-alkylated amino group, the alkyl substituents comprise 1 to 25, preferably 1 to 10 carbon atoms.

According to the present invention also pharmaceutically acceptable salts of a compound of formula (1) can be used for the formulation. According to the present invention also substances can be used which are metabolized to a compound as defined in formula (1) in the human organism or which lead to the release of a compound as defined in formula (1) for example by ester hydrolysis.

In a particular embodiment, the invention concerns the use of an a-carbon branched carboxylic acid as described in formula (1) or of a pharmaceutically acceptable salt thereof as an inhibitor of an enzyme having histone deacetylase activity and its use in the topical therapy of cancer, wherein R1 is a linear or branched, saturated or unsaturated, aliphatic C₂₋₂₅ hydrocarbon chain, R2 independently is a linear or branched, saturated or unsaturated, aliphatic C₂₋₂₅ hydrocarbon chain, but not -CH2-CH=CH2, -CH2-C≡CH or-CH2-CH2-CH3, R1 and R2 are optionally substituted with hydroxyl, amino, carboxylic, alkoxy, aryl and/or heterocyclic groups, and R3 is hydroxyl.

In yet another embodiment the invention concerns the use of an α-carbon branched carboxylic acid as described in formula (1) or of a pharmaceutically acceptable salt thereof for the topical therapy of cancer, wherein R1 is a linear or branched, saturated or unsaturated, aliphatic C₃₋₂₅ hydrocarbon chain, and R2 independently is a linear or branched, saturated or unsaturated, aliphatic C₃₋₂₅ hydrocarbon chain, R1 or R2 comprise one or several heteroatoms (e.g. O, N, S) replacing carbon atoms in the hydrocarbon chain, R1 and R2 are optionally substituted with hydroxyl, amino, carboxylic, alkoxy, aryl and/or heterocyclic groups, and R3 is hydroxyl.

In yet another embodiment of the invention R1 and R2 do not comprise an ester group (-CO-O-). R1 and R2 may be hydrocarbon chains comprising no heteroatoms O, N or S.

The compounds which are most preferably used according to the present invention are VPA and/or 4-yn VPA.

In a further aspect of the invention, other HDAC inhibitors are used.

Other active compounds which like VPA act as inhibitors of histone deacetylases may also be used in the same way to treat acne, in particular acne vulgaris, including but not limited to compounds and derivatives thereof such as LAQ824 (Novartis), LBH589 (Novartis), Trichostatin A, Suberoyl anilide hydroxamic acid (Aton/Merck), CBHA (Aton/Merck), Pyroxamide (Aton/Merck), Scriptaid (Johns Hopkins), CI-994 (Pfizer), CG-1521 (CircaGen), Chlamydocin (Janssen), Biaryl hydroxamate, e.g., A-161906 (Abbott), Bicyclic aryl-N-hydroxycarboxamides (Kansai University), PXD-101 (TopoTarget), MGCD0103 (MethylGene), TPX-HA analogue (CHAP) (Japan Energy), Oxamflatin, Trapoxin, Depudecin, Apidicin (Kyongji), benzamides such as MS-275 (Mitsui/Schering), pyroxamides and derivatives thereof, short chain fatty acids such as butyric acid, and derivatives thereof, e.g., Pivanex (Pivaloyloxymethyl butyrate), cyclic tetrapeptides such as trapoxin A, Depsipeptide (FK-228; Fujisawa/NCI) and related peptidic compounds, Tacedinaline (Pfizer), MG2856 (MethylGene), and HDAC class III inhibitors or SIRT inhibitors (see Kelly, O'Connor and Marks, 2002; Expert Opin. Investig. Drugs 11 (12), 1695-1713).

### Monotherapy and combination therapy

In one aspect, only one HDAC inhibitor is used in the treatment according to the invention, i.e. the treatment is a monotherapy. Preferably, VPA or a pharmaceutically acceptable derivative or prodrug therof is used as a single active agent in a monotherapy for the treatment of mild or moderate acne vulgaris.

In another aspect, the treatment according to the invention is a combination therapy, i.e. at least two different therapies are employed in the treatment of acne. Treatment with the HDAC inhibitor as described herein may be combined with another established principle of treating acne, e.g. with administration of antibiotics (systemic or topical), retinoids, topical bactericidals such as benzoyl peroxide, phototherapy or hormonal agents. Examples of topical antibiotics include erythromycin, clindamycin or tetracycline, examples of systemic antibiotics include erythromycin, tetracycline, oxytetracycline, doxycycline, minocycline and lymecycline. Examples of topical retinoids include tretinoin, adapalene, tazarotene and isotretinoin. Isotretinoin can also been applied orally. Examples of hormonal agents include oral contraceptives.

In yet another aspect, at least two different HDAC inhibitors are used in the combination treatment. The at least two different HDAC inhibitors are preferably selected from the HDAC inhibitors described supra.

In the case of a combination therapy, the different active agents can be administered simultaneously or sequentially, in any order. When the different active agents are administered simultaneously, they are usually comprised in the same composition. When the different active agents are administered sequentially, the period of time between administration of the first active agent and the second active agent may be up to 10 days, preferably up to 7 days, more preferably up to 5 days. This period of time may range from very short (1 minute or less) to 5 days, preferably it ranges from 1 hour to 3 days, most preferably from 6 hours to 2 days (e.g. about 24 hours).

The medicament may be applied once, twice, three times, four times or five times daily. Preferably, it is applied once to three times daily. The treatment may last at least 1 week preferably at least 2 weeks, more preferred at least 4 weeks, even more preferred at least 6 or at least 8 weeks.

The mean reduction of the number of acne lesions after 85 days of continuous treatment is usually at least 20 %, preferably at least 30 %, more preferably at least 40 %, most preferably at least 50 %, relative to the number of acne lesions at base line before start of the treatment.

The mean reduction of the number of inflammatory acne lesions after 85 days of continuous treatment is usually at least 20 %, preferably at least 30 %, more preferably at least 40 %, most preferably at least 50 %, relative to the number of inflammatory acne lesions at base line before start of the treatment.

The mean reduction of the number of non-inflammatory acne lesions after 85 days of continuous treatment is usually at least 20 %, preferably at least 30 %, more preferably at least 40 %, most preferably at least 50 %, relative to the number of non-inflammatory acne lesions at base line before start of the treatment.

The period of time within which a reduction of the total count by ≥30% of all acne lesions treated is observed in at least 50% of the patients treated is preferably less than 8 weeks, more preferably less than 6 weeks, more preferably less than 4 weeks, most preferably less than 3 weeks or less than 2 weeks. A reduction of the total counts by ≥30% of all acne lesions treated after 14 days of continuous treatment is usually observed in at least 10% of the patients treated, preferably in at least 20%, more preferably in at least 30%, most preferably in at least 40% or at least 50% of the patients treated.

The treatment usually causes signs of site irritation, site erythema or site pain in less than 75 % of the patients treated, preferably in less than 50 %, more preferably in less than 25%.

Another aspect of the invention is the use of VPA and/or pharmaceutically acceptable salts thereof, for the manufacture of a medicament for the prevention or treatment of acne wherein a medicament comprising at least about 0.1% VPA or derivative thereof is topically applied to the skin of an individual in need thereof.

VPA or its derivative may be used for the treatment of inflammations of the skin and/or mucosa, such as acne vulgaris, psoriasis or atopic dermatitis.

### Composition

The medicament which is to be topically applied is usually a pharmaceutical composition for the prevention or treatment of acne, comprising:
(i) at least about 0.1 % of an active agent selected from the group consisting of HDAC inhibitors, VPA and pharmaceutically acceptable salts thereof, derivatives and prodrugs of VPA and pharmaceutically acceptable salts thereof; and
(ii) a dermatologically acceptable carrier.

The term "topical application", as used herein, means to apply or spread the compositions of the present invention onto the surface of the skin.

The term "dermatologically acceptable," as used herein, means that the compositions or components thereof so described are suitable for use in contact with human skin without undue toxicity, incompatibility, instability, allergic response, and the like.

Unless indicated otherwise, percentage values given herein are % by weight (w/w).

The compositions of the present invention preferably comprise from about 0.1% to about 25%, more preferably from about 0.1 % to about 6%, even more preferably from about 0.3% to about 5%, still more preferably from about 0.5% to about 4%, still more preferably from about 1% to about 4%, most preferably from about 2% to about 4%, of the active agent.

The compositions of the present invention more preferably comprise VPA and/or pharmaceutically acceptable salts thereof, at a concentration of from about 0.1% to about 10%, more preferably from about 0.5% to about 6%, even more preferably from about 0.5% to about 4%, still more preferably from about 1% to about 4%, most preferably from about 2% to about 4%.

The compositions of the present invention usually comprise from about 1% to about 99.9% of a dermatologically acceptable carrier within which the compositions of the present invention is incorporated to enable the active agent, as well as other optional actives, to be delivered to the skin at an appropriate concentration.

In a preferred embodiment the composition of the invention is a semisolid at 25°C and under atmospheric pressure. In accordance with this embodiment, the product form of the composition may be a cream, an ointment, a gel or a paste. The product form of the composition may be a liquid dispersion, e.g. a lotion.

In a preferred embodiment, the carrier is not a solution. In another preferred embodiment, the carrier is a cream, a paste, an ointment, a lotion or a gel.

Another aspect of the invention is the use of VPA, a pharmaceutically acceptable salt thereof, a derivative of VPA or a pharmaceutically acceptable salt thereof for the manufacture of a medicament for the prevention or treatment of acne wherein a medicament comprising at least about 0.1% VPA or derivative thereof is topically applied to the skin of an individual in need thereof.

Further actives include inhibitors of histone deacetylases which are different to VPA, including but not limited to compounds such as NVP-LAQ824 (Novartis), LBH-589 (Novartis), Trichostatin A, Suberoyl anilide hydroxamic acid (Aton/Merck), CBHA (Aton/Merck), Pyroxamide (Aton/Merck), Scriptaid (Johns Hopkins), CI-994 (Pfizer), CG-1521 (CircaGen), Chlamydocin (Janssen), Biaryl hydroxamate, e.g., A-161906 (Abbott), Bicyclic aryl-N-hydroxycarboxamides (Kansai University), PXD-101 (TopoTarget), MGCD0103 (Methyl Gene), TPX-HA analogue (CHAP) (Japan Energy), Oxamflatin, Trapoxin, Depudecin, Apidicin (Kyongji), benzamides such as MS-275 (Mitsui/Schering), pyroxamides and derivatives thereof, short chain fatty acids such as butyric acid, and derivatives thereof, e.g., Pivanex (Pivaloyloxymethyl butyrate), cyclic tetrapeptides such as trapoxin A, Depsipeptide (FK- 228; Fujisawa/NCI) and related peptidic compounds, Tacedinaline (Pfizer), MG2856 (Methyl Gene), and HDAC class III inhibitors or SIRT inhibitors (see Kelly, O'Connor and Marks, 2002; Expert Opin. Investig. Drugs 11 (12), 1695-1713). differentiation inducing drugs (e.g. vitamin D, vitamin D derivatives, retinoids, receptor binding agents such as imiquimode), radiation therapy (e.g. x-rays or gamma rays), immunological approaches (antibody therapy, vaccination), combined immunotherapeutic/cytotoxic approaches (e.g. antibodies conjugated with cytotoxic components), and the like. These further actives can be used instead of VPA or in combination with VPA.

The invention further provides a topically applicable formulation of a medicament containing Valproic Acid or derivatives thereof used alone or in combination with retinoids, for the topical treatment of acne. Accordingly, one aspect of the present invention is the use of a topically applicable formulation containing VPA or derivatives thereof used alone or in combination with retinoids for a topical treatment of acne The anti-acne activity of combinatorial topical treatments compared to the use of each component alone can thus be increased and -if desired -the doses of the individual components of such combinatorial treatments may be lowered in order to decrease unwanted side effects related to treatment with the individual drugs alone.

### Formulation

The compositions of the present invention comprise from about 1 % to about 99.9% of a dermatologically acceptable carrier within which the compositions of the present invention is incorporated to enable the active agent, as well as other optional actives, to be delivered to the skin at an appropriate concentration. The carrier may contain one or more dermatologically acceptable solid, semi- solid or liquid fillers, diluents, solvents, extenders and the like. The carrier may be solid, semi-solid or liquid. Preferred carriers are substantially semi-solid. The carrier can itself be inert or it can possess dermatological benefits of its own. Concentrations of the carrier can vary with the carrier selected and the intended concentrations of the active agent and optional components.
Suitable carriers include conventional or otherwise known carriers that are dermatologically acceptable. The carrier should also be physically and chemically compatible with the essential components described herein, and should not unduly impair stability, efficacy or other use benefits associated with the compositions of the present invention. Preferred components of the compositions of this invention should be capable of being coming led in a manner such that there is no interaction which would substantially reduce the efficacy of the composition under ordinary use situations. The type of carrier utilized in the present invention depends on the type of product form desired for the composition. The topical compositions useful in the subject invention may be made into a wide variety of product forms such as are known in the art. These include, but are not limited to, lotions, creams, gels, sticks, sprays, ointments, oils, foams, powders and pastes. These product forms may comprise several types of carriers, including, but not limited to, solutions, aerosols, emulsions, gels, solids, and liposomes. Preferred carriers contain a dermatologically acceptable, hydrophilic diluent. As used herein, "diluent" includes materials in which the particulate material can be dispersed, dissolved, or otherwise incorporated. Non-limiting examples of hydrophilic diluents are water, organic hydrophilic diluents such as lower monovalent alcohols (e.g., C1-C4) and low molecular: weight glycols and polyols, including propylene glycol, polyethylene glycol (e.g., Molecular Weight 200-600 g/mole), polypropylene glycol (e.g., Molecular Weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Water is a preferred diluent. The composition preferably comprises from about 60% to about 99.99% of the hydrophilic diluent.

Solutions according to the subject invention typically include a dermatologically acceptable hydrophilic diluent. Solutions useful in the subject invention preferably contain from about 60% to about 99.99% of the hydrophilic diluent.

Aerosols according to the subject invention can be formed by adding a propellant to a solution such as described above. Exemplary propellants include chloro-fluorinated lower molecular weight hydrocarbons. Aerosols are typically applied to the skin as a spray-on product.

Preferred carriers comprise an emulsion comprising a hydrophilic phase comprising a hydrophilic component, e.g., water or other hydrophilic diluent, and a hydrophobic phase comprising a hydrophobic component, e.g., a lipid, oil or oily material. As well known to one skilled in the art, the hydrophilic phase will be dispersed in the hydrophobic phase, or vice versa, to form respectively hydrophilic or hydrophobic dispersed and continuous phases, depending on the composition ingredients. In emulsion technology, the term "dispersed phase" is a term well-known to one skilled in the art which means that the phase exists as small particles or droplets that are suspended in and surrounded by a continuous phase. The dispersed phase is also known as the internal or discontinuous phase. The emulsion may be or comprise (e.g., in a triple or other multi- phase emulsion) an oil-in-water emulsion or a water-in-oil emulsion such as a water-in- silicone emulsion. Oil-in-water emulsions typically comprise from about 1% to about 50% of the dispersed hydrophobic phase and from about 1% to about 98% of the continuous hydrophilic phase; water-in-oil emulsions typically comprise from about 1% to about 98% of the dispersed hydrophilic phase and from about 1% to about 50% of the continuous hydrophobic phase. The emulsion may also comprise a gel network. Preferred emulsions are further described below.

The topical compositions of the subject invention, including but not limited to lotions and creams, may comprise a dermatologically acceptable emollient. Such compositions preferably contain from about 2% to about 50% of the emollient.

Emollients tend to lubricate the skin, increase the smoothness and suppleness of the skin, prevent or relieve dryness of the skin, and/or protect the skin. Emollients are typically water-immiscible, oily or waxy materials. Non-limiting examples of emollients are described herein.

Lotions and creams according to the present invention preferably comprise a solution carrier system and one or more emollients. Lotions typically comprise from about 1% to about 20%, preferably from about 5% to about 10%, of emollient; from about 50% to about 90%, preferably from about 60% to about 80%, water. A cream typically comprises from about 5% to about 50%, preferably from about 10% to about 20%, of emollient; and from about 45% to about 85%, preferably from about 50% to about 75%, water.

Ointments of the present invention may comprise a simple carrier base of animal or vegetable oils or semi-solid hydrocarbons (oleaginous); absorption ointment bases which absorb water to form emulsions; or water soluble carriers, e.g., a water soluble solution carrier. Ointments may further comprise a thickening agent. For example, an ointment may comprise from about 2% to about 10% of an emollient; and from about 0.1 % to about 2% of a thickening agent. Non-limiting examples of thickening agents are described herein.

Preferred topical compositions of the present invention comprise an emulsion. Emulsions of the present invention may contain one or more of the following:
a) Hydrophobic component
   Emulsions according to the present invention contain a hydrophobic phase comprising a lipid, oil, oily or other hydrophobic component. The compositions of the present invention preferably comprise from about 1% to about 50%, preferably from about 1% to about 30%, and more preferably from about 1% to about 10% by weight of the composition of a hydrophobic component. The hydrophobic component may be derived from animals, plants, or petroleum and may be natural or synthetic (i.e., man-made). Preferred hydrophobic components are substantially water-insoluble, more preferably essentially water-insoluble. Nonlimiting examples of suitable hydrophobic components include those selected from the group consisting of:
   (1) Mineral oil, which is also known as petrolatum liquid, is a mixture of liquid hydrocarbons obtained from petroleum. See The Merck Index, Tenth Edition, Entry 7048, p. 1033 (1983).
   (2) Petrolatum, which is also known as petroleum jelly, is a colloidal system of nonstraight-chain solid hydrocarbons and high-boiling liquid hydrocarbons, in which most of the liquid hydrocarbons are held inside the micelles. See The Merck Index, Tenth Edition, Entry 7047, p. 1033 (1983); Schindler, Drug. Cosmet. Ind., 89, 36-37,76,78-80, 82 (1961).
   (3) Straight and branched chain hydrocarbons having from about 7 to about 40 carbon atoms. Nonlimiting examples of these hydrocarbon materials include dodecane, isododecane, squalane, cholesterol, hydrogenated polyisobutylene, docosane, hexadecane, isohexadecane. Also useful are the C7-C40 isoparaffins, which are C7-C40 branched hydrocarbons, e.g., C13-C14 isoparaffin.
   (4) C1-C30 alcohol esters of C1-C30 carboxylic acids and of C2-C30 dicarboxylic acids, including straight and branched chain materials as well as aromatic derivatives (as used herein in reference to the hydrophobic component, mono- and poly- carboxylic acids include straight chain, branched chain and aryl carboxylic acids). Nonlimiting examples include diisopropyl sebacate, diisopropyl adipate, isopropyl myristate, isopropyl palmitate, methyl palmitate, myristyl propionate, 2-ethylhexyl palmitate, isodecyl neopentanoate, di-2-ethylhexyl maleate, cetyl palmitate, myristyl myristate, stearyl stearate, isopropyl stearate, methyl stearate, cetyl stearate, behenyl behenrate, dioctyl maleate, dioctyl sebacate, diisopropyl adipate, cetyl octanoate, diisopropyl dilinoleate.
   (5) mono-, di- and tri-glycerides of C1-C30 carboxylic acids, e.g., caprilic/capric triglyceride, PEG-6 caprylic/capric triglyceride, PEG-8 caprylic/capric triglyceride.
   (6) alkylene glycol esters of C1-C30 carboxylic acids, e.g., ethylene glycol mono- and di-esters, and propylene glycol mono- and di-esters of C1-C30 carboxylic acids e.g., ethylene glycol distearate.
   (7) propoxylated and ethoxylated derivatives of the foregoing materials.
   (8) C1-C30 mono- and poly-esters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a 1:2 molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 2:6 molar ratio; and the octaester of sucrose wherein the esterifying carboxylic acid moieties are laurate, linoleate and behenate in a 1:3.4 molar ratio. A preferred solid material is sucrose polyester in which the degree of esterification is 7-8, and in which the fatty acid moieties are C 18 mono- and/or di-unsaturated and behenic, in a molar ratio of unsaturates:behenic of 1:7 to 3:5. A particularly preferred solid sugar polyester is the octaester of sucrose in which there are about 7 behenic fatty acid moieties and about 1 oleic acid moiety in the molecule. Other materials include cottonseed oil or soybean oil fatty acid esters of sucrose.
   (9) Organopolysiloxane oils. The organopolysiloxane oil may be volatile, non- volatile, or a mixture of volatile and non-volatile silicones. The term "nonvolatile" as used in this context refers to those silicones that are liquid under ambient conditions. The term "volatile" as used in this context refers to all other silicone oils. Suitable organopolysiloxanes can be selected from a wide variety of silicones spanning a broad range of volatilities and viscosities. Nonvolatile polysiloxanes are preferred. Examples of suitable organopolysiloxane oils include polyalkylsiloxanes, cyclic polyalkylsiloxanes, and polyalkylarylsiloxanes. Preferred for use herein are organopolysiloxanes selected from the group consisting of polyalkylsiloxanes, alkyl substituted dimethicones, cyclomethicones, trimethylsiloxysilicates, dimethiconols, polyalkylaryl siloxanes, and mixtures thereof. More preferred for use herein are polyalkylsiloxanes and cyclomethicones. Preferred among the polyalkylsiloxanes are dimethicones, as described in US 5,968,528.
   (10) Vegetable oils and hydrogenated vegetable oils. Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated seSan1e oil, hydrogenated sunflower seed oil, and mixtures-thereof.
   (11) animal fats and oils e.g., lanolin and derivatives thereof, cod liver oil.
   (12) Also useful are C4-C20 alkyl ethers of polypropylene glycols, C1-C20 carboxylic acid esters of polypropylene glycols, and di-C8-C30 alkyl ethers. Nonlimiting examples of these materials include PPG-14 butyl-ether, PPG-15 stearyl ether, dioctyl ether, dodecyl octyl ether, and mixtures thereof.
b) Hydrophilic component
   Emulsions of the present invention also comprise a hydrophilic component, e.g., water or other hydrophilic diluent. The hydrophilic phase can thus comprise water, or a combination of water and one or more water soluble or dispersible ingredients. Hydrophilic components comprising water are preferred.
c) Other components
   Emulsions and other topical compositions of the present invention may comprise a variety of other ingredients such as disclosed herein. As will be understood by the skilled artisan, a given component will distribute primarily into either a hydrophilic phase or hydrophobic phase, depending on the hydrophilicity of the component in the composition.
   Emulsions of the present invention preferably include one or more compounds selected from emulsifiers, surfactants, structuring agents, and thickeners.

### (1) Emulsifiers/Surfactants

The emulsion may contain an emulsifier and/or surfactant, generally to help disperse and suspend the discontinuous phase within the continuous phase. A wide variety of such agents can be employed. Known or conventional emulsifiers/surfactants can be used in the composition, provided that the selected agent is chemically and physically compatible with essential components of the composition, and provides the desired dispersion characteristics. Suitable agents include non-silicone-containing emulsifiers/surfactants, silicone emulsifiers/surfactants, and mixtures thereof.

In a preferred embodiment, the composition comprises a hydrophilic emulsifier or surfactant. The compositions of the present invention preferably comprise from about 0.05% to about 5%, more preferably from about 0.05% to about 1% of at least one r hydrophilic surfactant.

Preferred hydrophilic surfactants are selected from nonionic surfactants. Among the nonionic surfactants that are useful herein are those that can be broadly defined as condensation products of long chain alcohols, e.g. C8-30 alcohols, with sugar or starch polymers, i.e., glycosides. Examples of long chain alcohols from which the alkyl group can be derived include decyl alcohol, cetyl alcohol, stearyl alcohol, lauryl alcohol, myristyl alcohol, oleyl alcohol, and the like. Commercially available examples of these surfactants include decyl polyglucoside (available as APG 325 CS from Henkel) and lauryl polyglucoside (available as APG 600 CS and 625 CS from Henkel).

Other useful nonionic surfactants include the condensation products of alkylene oxides with fatty acids (i.e. alkylene oxide esters of fatty acids). Other nonionic surfactants are the condensation products of alkylene oxides with fatty alcohols (i.e. alkylene oxide; ethers of fatty alcohols). Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-I0 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl coco ate, PEG-200 glyceryl tallowate, PEG-8 dilaurate, PEG-10 distearate, and mixtures thereof.

Still other useful non ionic surfactants include polyhydroxy fatty acid amide surfactants.

Preferred among the nonionic surfactants are those selected from the group consisting of steareth-21, ceteareth-20, ceteareth-12, sucrose cocoate, steareth-100, PEG-100 stearate, and mixtures thereof.

Other nonionic surfactants suitable for use herein include sugar esters and polyesters, alkoxylated sugar esters and polyesters, C1-C30 fatty acid esters of C1-C30 (fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-30 ethers of polyols, alkyl phosphates, polyoxyalkylene fatty ether phosphates, fatty acid -amides, acyllactylates, and mixtures thereof. Nonlimiting examples of these non-silicon-contairiing emulsifiers include: polyethylene glycol 20 sorbitan monolaurate (Polysorbate 20), polyethylene glycol 5, soya sterol, Steareth-20, Ceteareth-20, PPG-2 methyl glucose ether distearate, Ceteth-10, Polysorbate 80, cetyl phosphate, potassium cetyl phosphate, diethanolamine, cetyl phosphate, Polysorbate 60, glyceryl stearate, polyoxyethylene 20 sorbitan trioleate (Polysorbate 85), sorbitan monolaurate, polyoxyethylene 4 lauryl ether, sodium stearate, polyglyceryl-4 isostearate, hexyllaurate, PPG-2 methyl glucose ether distearate, PEG-100 stearate, and mixtures thereof.

Another emulsifier useful herein are fatty acid ester blends based on a mixture of sorbitan or sorbitol fatty acid ester and sucrose fatty acid ester. The preferred fatty acid ester emulsifier is a blend of sorbitan or sorbitol C16-C20 fatty acid ester with sucrose C10- C16 fatty acid ester, especially sorbitan stearate and sucrose cocoate. This is commercially available from ICI under the trade name Arlatone 2121.

The hydrophilic surfactants useful herein can alternatively or additionally include any of a wide variety of cationic, anionic, zwitterionic, and amphoteric surfactants such as are known in the art. See, e.g., McCutcheon's, Detergents and Emulsifiers, North American Edition (1986), published by Allured Publishing Corporation.

Exemplary cationic surfactants useful herein include those disclosed in U.S. Pat. No. 5,968,528. The cationic surfactants useful herein include cationic ammonium salts such as quaternary ammonium salts, and amino-amides.

A wide variety of anionic surfactants are also useful herein. See e.g., US. Pat. No. 5,968,528. Nonlimiting examples of anionic surfactants include the alkoyl isethionates (e.g., C12-C30), alkyl and alkyl ether sulfates and salts thereof, alkyl and alkyl ether phosphates and salts thereof, alkyl methyl taurates (e.g., C12 -C30), and soaps (e.g. alkali metal salts, e.g., sodium or potassium salts) of fatty acids.

Amphoteric and zwitterionic surfactants are also useful herein. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C8-C18) and one contains an anionic water solubilizing group, e.g., carboxy, sulfonate, sulfate, phosphate, or phosphonate. Examples are alkyl imino acetates, and iminodialkanoates and aminoalkanoates, imidazolinium and ammonium derivatives. Other suitable amphoteric and zwitterionic surfactants are those selected from the group consisting of betaines, sultaines, hydroxysultaines, alkyl sarcosinates (e.g., C12-C30), and alkanoyl sarcosinates.

Preferred emulsions of the present invention include a silicone containing emulsifier or surfactant. A wide variety of silicone emulsifiers are useful herein. These silicone emulsifiers are typically organically modified organopolysiloxanes, also known - to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds which contain C2-C30 pendant side chains. Still other useful dimethicone copolyols include materials having various cationic, anionic, amphoteric, and zwitterionic pendant moieties.

Nonlimiting examples of dimethicone copolyols and other silicone surfactants useful as emulsifiers herein include polydimethylsiloxane polyether copolymers with pendant polyethylene oxide sidechains, polydimethylsiloxane polyether copolymers with pendant polypropylene oxide sidechains, polydimethylsiloxane polyether copolymers with pendant mixed polyethylene oxide and polypropylene oxide sidechains, polydimethylsiloxane polyether copolymers with pendant mixed poly(ethylene)(propylene)oxide sidechains, polydimethylsiloxane polyether copolymers with pendant organobetaine sidechains, polydimethylsiloxane polyether copolymers with pendant carboxylate sidechains, polydimethylsiloxane polyether copolymers with pendant quaternary ammonium sidechains; and also further modifications of the preceding copolymers containing pendant C2-C30 straight, branched, or cyclic alkyl moieties. Examples of commercially available dimethicone copolyols useful herein sold by Dow Coming Corporation are Dow Corning^{®} 190, 193, Q2-5220, 2501 Wax, 2-5324 fluid, and 3225C (this later material being sold as a mixture with cyclomethicone). Cetyl dimethicone copolyol is commercially available as a mixture with polyglyceryl-4 isostearate (and) hexyllaurate and is sold under the tradename ABIL^{®} WE-O9 (available from Goldschmidt). Cetyl dimethicone copolyol is also commercially available as a mixture with hexyllaurate (and) polyglyceryl-3 oleate (and) cetyl dimethicone and is sold under the tradename ABIL^{®} WS-08 (also available from Goldschmidt). Other nonlimiting examples of dimethicone copolyols also include lauryl dimethicone copolyol, dimethicone copolyol acetate, dimethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyollaurate, dimethicone copolyol methyl ether, dimethicone copolyol phosphate, and dimethicone copolyol stearate. Dimethicone copolyol emulsifiers useful herein are described, for example, in U.S. Pat. No. 5,968,528.

### (2) Structuring Agent

The compositions hereof, and especially the emulsions hereof, may contain a structuring agent. Structuring agents are particularly preferred in the oil-in-water emulsions of the present invention. Without being limited by theory, it is believed that the structuring agent assists in providing rheological characteristics to the composition which contribute to the stability of the composition. For example, the structuring agent tends to assist in the formation of the liquid crystalline gel network structures. The structuring agent may also function as an emulsifier or surfactant. Preferred compositions of this invention comprise from about 1% to about 20%, more preferably from about 1% to about 10%, most preferably from about 2% to about 9%, of one or more structuring agents. Preferred structuring agents are those having an HLB of from about 1 to about 8 and having a melting point of at least about 45°C. Suitable structuring agents are those selected from the group consisting of saturated C14 to C30 fatty alcohols, saturated C16 to C30 fatty alcohols containing from about 1 to about 5 moles of ethylene oxide, saturated C16 to C30 diols, saturated C16 to C30 monoglycerol ethers, saturated C16 to C30 hydroxy fatty acids, C14 to C30 hydroxylated and nonhydroxylated saturated fatty acids, C14 to C30 saturated ethoxylated fatty acids, amines and alcohols containing from about 1 to about 5 moles of ethylene oxide diols, C14 to C30 saturated glyceryl mono esters with a monoglyceride content of at least 40%, C14 to C30 saturated polyglycerol esters having from about 1 to about 3 alkyl groups, from about 2 to about 3 saturated glycerol units, C14 to C30 glyceryl mono ethers, C14 to C30 sorbitan mono/diesters, C14 to C30 saturated ethoxylated sorbitan mono/diesters with about 1 to about 5 moles of ethylene oxide, C14 to C30 saturated methyl glucoside esters, C14 to C30 saturated sucrose mono/diesters, C14 to C30 saturated ethoxylated methyl glucoside esters with about 1 to about 5 moles of ethylene oxide, C14 to C30 saturated polyglucosides having an average of between 1 to 2 glucose units and mixtures thereof, having a melting point of at least about 45°C.

The preferred structuring agents of the present invention are selected from the group consisting of stearic acid, palmitic acid, stearyl alcohol, cetyl alcohol, behenyl alcohol, stearic acid, palmitic acid, the polyethylene glycol ether of stearyl alcohol having an average of about 1 to about 5 ethylene oxide units, the polyethylene glycol ether of cetyl alcohol having an average of about 1 to about 5 ethylene oxide units, and mixtures thereof. More preferred structuring agents of the present invention are selected from the group consisting of stearyl alcohol, cetyl alcohol, behenyl alcohol, the polyethylene glycol ether of stearyl alcohol having an average of about 2 ethylene oxide units (steareth-2), the polyethylene glycol ether of cetyl alcohol having an average of about 2 ethylene oxide units, and mixtures thereof. Even more preferred structuring agents are selected from the group consisting of stearic acid, palmitic acid, stearyl alcohol, cetyl alcohol, behenyl alcohol, steareth-2, and mixtures thereof.

### (3) Thickening Agent (Including Thickeners and Gelling Agents)

The compositions of the present invention can also comprise a thickening agent, preferably from about 0.1 % to about 5%, more preferably from about 0.1 % to about 3%, and most preferably from about 0.25% to about 2%, of a thickening agent. Nonlimiting classes of thickening agents include those selected from the group consisting of:
(i) Carboxylic Acid Polymers
   These polymers are crosslinked compounds containing one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. The preferred carboxylic acid polymers are of two general types. The first type of polymer is a crosslinked homopolymer of an acrylic acid monomer or derivative thereof (e.g., wherein the acrylic acid has substituents on the two and three carbon positions independently selected from the group consisting of C1-4 alkyl, -CN, -COOH, and mixtures thereof). The second type of polymer is a crosslinked copolymer having a first monomer selected from the group consisting of an acrylic acid monomer or derivative thereof (as just described in the previous sentence), a short chain alcohol (i.e., a C 1-4) acrylate ester monomer or derivative thereof (e.g., wherein the acrylic acid portion of the ester has substituents on the two and three carbon positions independently selected from the group consisting of C1-4 alkyl, -CN, -COOH, and mixtures thereof), and mixtures thereof; and a second monomer which is a long chain alcohol (i.e. C8-40) acrylate ester monomer or derivative thereof (e.g., wherein the acrylic acid portion of the ester has substituents on the two and three carbon positions independently selected from the group consisting of C1-4 alkyl, -CN, -COOH, and mixtures thereof). Combinations of these two types of polymers are also useful herein.
   In the first type of crosslinked homopolymers, the monomers are preferably selected from the group consisting of acrylic acid, methacrylic acid, ethacrylic acid, and mixtures thereof, with acrylic acid being most preferred. In the second type of crosslinked copolymers the acrylic acid monomer or derivative thereof is preferably selected from the group consisting of acrylic acid, methacrylic acid, ethacrylic acid, and mixtures thereof, with acrylic acid, methacrylic acid, and mixtures thereof being most preferred. The short chain alcohol acrylate estermonomer or derivative thereof is preferably selected from the group consisting of C1-4 alcohol acrylate esters, C1-4 alcohol methacrylate esters, C1-4 alcohol ethacrylate esters, and mixtures thereof, with the C1-4 alcohol acrylate esters, C1-4 alcohol methacrylate esters, and mixtures thereof, being most preferred. The long chain alcohol acrylate ester monomer is selected from C8-40 alkyl acrylate esters, with C10-30 alkyl acrylate esters being preferred.
   The crosslinking agent in both of these types of polymers is polyalkenyl polyether of a polyhydric alcohol containing more than one alkenyl ether group per molecule, wherein the parent polyhydric alcohol contains at least 3 carbon atoms and at least 3 hydroxyl groups. Preferred crosslinkers are those selected from the group consisting of allyl ethers of sucrose and allyl ethers of pentaerythritol, and mixtures thereof.
   Examples of commercially available homopolymers of the first type useful herein include the carbomers, which are homopolymers of acrylic acid crosslinked with allyl ethers of sucrose or pentaerytritol. The carbomers are available as the Carbopol^{®} 900 series from B.F. Goodrich (e.g., Carbopol^{®} 954). Examples of commercially available copolymers of the second type useful herein include copolymers of C10-30 alkyl acrylates with one or more monomers of acrylic acid, methacrylic acid, or one of their short chain (i.e. C1-4 alcohol) esters, wherein the crosslinking agent is an ally ether of sucrose or pentaerytritol. These copolymers are known as acrylates C10-30 alkyl acrylate crosspolymers and are commercially available as Carbopol^{®}1342, Carbopol^{®} 1382Pemulen TR-I, and Pemulen TR-2, from B. F. Goodrich. In other words, examples of carboxylic acid polymer thickeners useful herein are those selected from the group consisting of carbomers, acrylates/C10-C30 alkyl acrylate crosspolymers, and mixtures thereof.
(ii) Crosslinked Polyacrylate Polymers
   The crosslinked polyacrylate polymers useful as thickeners or gelling agents include both cationic and nonionic polymers, with the cationics being generally preferred. Non-limiting examples of suitable crosslinked polyacrylate polymers are disclosed in US 5,968,528.
(iii) Polyacrylamide Polymers
   Also useful herein are polyacrylamide polymers, especially non-ionic polyacrylamide polymers including substituted branched or unbranched polymers. These polymers can be formed from a variety of monomers including acrylamide and methacrylamide which are unsubstituted or substituted with one or two alkyl groups (preferably C1 to C5). Preferred are acrylate amide and methacrylate amide monomers in which the amide nitrogen is unsubstituted, or substituted with one or two C1 to C5 alkyl groups (preferably methyl, ethyl, or propyl), for example, acrylamide, methacrylamide, N-methacrylamide, N-methylmethacrylamide, N,N-dimethylmethacrylamide, N- isopropylacrylamide, N-isopropylmethacrylamide, and N,N-dimethylacrylamide. These polymers have a molecular weight greater than about 1,000,000 preferably greater than about 1,5000,000 and range up to about 30,000,000. Most preferred among these polyacrylamide polymers is the non-ionic polymer given the CTF A designation polyacrylamide and isoparaffin and laureth-7, available under the Tradename Sepigel 305 from Seppic Corporation (Fairfield, N.J.).
   Other polyacrylamide polymers useful herein include multi-block copolymers of acrylamides and substituted acrylamides with acrylic acids and substituted acrylic acids. Commercially available examples of these multi-block copolymers include Hypan SR150H, SS500V, SS500W, SSSA100H, from Lipo Chemicals, Inc., (Patterson, N.J.).
(iv) Polysaccharides
   A wide variety of polysaccharides are useful herein. By "polysaccharides" are meant gelling agents containing a backbone of repeating sugar (i.e. carbohydrate) units. Non-limiting examples of polysaccharide gelling agents include those selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also useful herein are the alkyl substituted celluloses. In these polymers, the hydroxy groups of the cellulose polymer is hydroxyalkylated (preferably hydroxyethylated or hydroxypropylated) to form a hydroxyalkylated cellulose which is then further modified with a C10-C30 straight chain or branched chain alkyl group through an ether linkage. Typically these polymers are ethers of C10-C30 straight or branched chain alcohols with hydroxyalkylcelluloses. Examples of alkyl groups useful herein include those selected from the group consisting of stearyl, isostearyl, lauryl, myristyl, cetyl, isocetyl, cocoyl (i.e. alkyl groups derived from the alcohols of coconut oil), palmityl, oleyl, linoleyl, linolenyl, ricinoleyl, behenyl, and mixtures thereof. Preferred among the alkyl hydroxyalkyl cellulose ethers is the material given the CTFA designation cetyl hydroxyethylcellulose, which is the ether of cetyl alcohol and hydroxyethylcellulose. This material is sold under the tradename Natrosol^{®} CS Plus from Aqualon Corporation.
(v) Gums
   Other additional thickening and gelling agents useful herein include materials which are primarily derived from natural sources. Non-limiting examples of these gelling agent gums include materials selected from the group consisting of acacia, agar, algin, alginic acid, ammonium alginate, amylopectin, calcium alginate, calcium carrageenan, camitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.
(vi) Crosslinked Vinyl Ether Maleic Anhydride Copolymers
   Other additional thickening and gelling agents useful herein include crosslinked copolymers of alkyl vinyl ethers and maleic anhydride.
(vii) Crosslinked Poly(N-vinylpyrrolidones)
   Crosslinked polyvinyl(N-pyrrolidones) useful herein as additional thickening and gelling agents These gelling agents typically contain from about 0.25% to about 1% by weight of a crosslinking agent selected from the group consisting of divinyl ethers and diallyl ethers of terminal diols containing from about 2 to about 12 carbon atoms, divinyl ethers and diallyl ethers of polyethylene glycols containing from about 2 to about 600 units, dienes having from about 6 to about 20 carbon atoms, divinyl benzene, vinyl and allyl ethers of pentaerythritol, and the like.

Preferred compositions of the present invention include a thickening agent selected from the group consisting of carboxylic acid polymers, crosslinked polyacrylate polymers, polyacrylamide polymers, and mixtures thereof, more preferably selected from the group consisting of crosslinked polyacrylate polymers, polyacrylamide polymers, and mixtures thereof.

### Optional Components

The topical compositions of the present invention may comprise a wide variety of optional components, provided that such optional components are physically and chemically compatible with the essential components described herein, and do not unduly impair stability, efficacy or other use benefits associated with the compositions of the present invention. Optional components may be dispersed, dissolved or the like in the carrier of the present compositions.

Optional components include aesthetic agents and active agents. For example, the compositions may include, in addition to the essential components of the invention, absorbents (including oil absorbents such as clays and polymeric absorbents), abrasives, anti caking agents, antifoaming agents, antimicrobial agents (e.g., a compound capable of destroying microbes, preventing the development of microbes or preventing the pathogenic action of microbes and useful, for example, in controlling acne and/or preserving the topical composition), binders, biological additives, buffering agents, bulking agents, chemical additives, cosmetic biocides, denaturants, cosmetic astringents, drug astringents, external analgesics, film formers, humectants, opacifying agents, fragrances, perfumes, pigments, colorings, essential oils, skin sensates, emollients, skin soothing agents, skin healing agents, pH adjusters, plasticizers, preservatives, preservative enhancers, propellants, reducing agents, skin-conditioning agents, skin penetration enhancing agents, skin protectants, solvents, suspending agents, emulsifiers, thickening agents, solubilizing agents, polymers for aiding the film-forming properties and substantivity of the composition (such as a copolymer of eicosene and vinyl pyrrolidone, an example of which is available from GAF Chemical Corporation as Ganex^{®} V -220), waxes, sunscreens, sunblocks, ultraviolet light absorbers or scattering agents, sunless tanning agents, antioxidants and/or radical scavengers, chelating agents, sequestrants, anti-acne agents, anti-inflammatory agents, anti-androgens, depilation agents, desquamation agents/exfoliants, organic hydroxy acids, vitamins and derivatives thereof (including water dispersible or soluble vitamins such as Vitamin C and ascorbyl phosphates), compounds which stimulate collagen production, and natural extracts. Such other materials are known in the art. Nonexclusive examples of such materials are described in Pharmaceutical Dosage Forms-Disperse Systems; Lieberman, Rieger & Banker, Vols. 1. (1988) & 2 (1989); or in US 5,968,528.

The composition of the invention may comprise an emollient. The emollient may be selected from one or more of the following classes: Triglyceride esters which include, but are not limited to, vegetable and animal fats and oils such as castor oil, cocoa butter, safflower oil, cottonseed oil, corn oil, olive oil, cod liver oil, almond oil, avocado oil, palm oil, sesame oil, squalene, kikui oil and soybean oil; Acetoglyceride esters, such as acetylated monoglycerides; Ethoxylated glycerides, such as ethoxylated glyceryl monostearate; Alkyl esters of fatty acids having 10 to 20 carbon atoms which include, but are not limited to, methyl, isopropyl, and butyl esters of fatty acids such as hexyllaurate, isohexyllaurate, isohexyl palmitate, isopropyl palmitate, methyl palmitate, decyloleate, isodecyl oleate, hexadecyl stearate decyl stearate, isopropyl isostearate, methyl isostearate, diisopropyl adipate, diisohexyl adipate, dihexyldecyl adipate, diisopropyl sebacate, lauryllactate, myristyllactate, and cetyl lactate; Alkenyl esters of fatty acids having 10 to 20 carbon atoms such as oleyl myristate, oleyl stearate, and oleyl oleate; Fatty acids having 10 to 20 carbon atoms such as pelargonic, lauric, myristic, palmitic, stearic, isostearic, hydroxystearic, oleic, linoleic, ricinoleic, arachidic, behenic, and erucic acids; Fatty alcohols having 10 to 20 carbon atoms such as lauryl, myristyl, cetyl, hexadecyl, stearyl, isostearyl, hydroxystearyl, oleyl, ricinoleyl, behenyl, erucyl, and 2- octyl dodecanyl alcohols; Lanolin and lanolin derivatives such as lanolin, lanolin oil, lanolin wax, lanolin alcohols, lanolin fatty acids, isopropyllanolate, ethoxylated cholesterol, propoxylated lanolin alcohols, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, and liquid and semisolid lanolin absorption bases; Polyhydric alcohol esters such as ethylene glycol mono and di-fatty acid esters, diethylene glycol mono-and di-fatty acid esters, polyethylene glycol (200-6000) mono- and di-fatty acid esters, propylene glycol mono- and di-fatty acid esters, polypropylene glycol 2000 monooleate, polypropylene glycol 2000, monostearate, ethoxylated propylene glycol monostearate, glyceryl mono- and di-fatty acid esters, polyglycerol polyfatty esters, ethoxylated glyceryl monostearate, 1,2-butylene glycol monostearate, 1,2-butylene glycol distearate, sorbitan fatty acid esters, and polyoxyethylene sorbitan fatty acid esters; Wax esters such as beeswax, spermaceti, myristyl myristate, stearyl stearate; Beeswax derivatives such as polyoxyethylene sorbitol beeswax which are reaction products of beeswax with ethoxylated sorbitol of varying ethylene oxide content, forming a mixture of ether esters; Vegetable waxes including, but not limited to, carnauba and candelilla waxes; Phospholipids such as lecithin and derivatives; Sterols including, but not limited to, cholesterol and cholesterol fatty acid esters; and amides such as fatty acid amides, ethoxylated fatty acid amides, and solid fatty acid alkanolamides.

The composition may comprise humectants, e.g., of the polyhydric alcohol- type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexanetriol (e.g., 1,2,6- hexanetriol), glycerol, ethoxylated glycerol, propoxylated glycerol, sodium 2-pyrrolidone-5-carboxylate, soluble collagen, dibutyl phthalate, gelatin and mixtures thereof.

Further optional components are guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g., aloe vera gel); sugar and starch derivatives (e.g., alkoxylated glucose); hyaluronic acid and derivatives thereof (e.g., salt derivatives such as sodium hyaluraonate); lactamide monoethanolamine; acetamide monoethanolamine; urea; panthenol; sugars; starches; silicone fluids; silicone gums; and mixtures thereof. Also useful are the propoxylated glycerols.

### Preferred embodiments:

Both hydrophilic and lipophilic creams were investigated. These creams are made up of highly biocompatible ingredients. In particular, the lipid phases of these cream systems may be made up of any vegetal oil, such as olive oil, sweet almond oil, avocado oil, jojoba oil and others, of mineral oils, such as vaseline or others, and/or of any synthetic oil phase, such as esters of fatty acids, short, medium and long chain triglycerides. To prepare these systems dermatologically acceptable surfactants that are able to provide a certain physicochemical stability to creams may be used, e.g. lecithin, Tween 80 and the like. Gel-based formulations were also investigated as potential formulations for the topical application of valproic acid, its salts and derivatives. Gels can be prepared by using any viscosizing agent that is able to provide the desired rheological features to formulations. Also in this case, agents to be used for the preparation of gel- based formulations must have a certain human skin tolerability, i.e. sepigel, zilgel, carbopol, xanthan gum, gelatin, PVP, PEa and the like.

### Dosing

Specific topical dose levels for any particular patient may be employed depending upon a variety of factors including the age, body weight, general health, sex, diet, and prior medication, and the severity of the particular disease of the patient, and the activity of specific compounds employed, time of administration, rate of excretion, the duration of the treatment, other drugs, compounds, and/or materials used in combination. It will be appreciated that the appropriate dosage of the active compounds, and compositions comprising the active compounds, can vary from patient to patient. Determining the optimal dosage will generally involve balancing of the level of therapeutic benefit against any risk or deleterious side effects of the treatments of the present invention.

In general, a suitable dose of the active compound, i.e., VPA or pharmaceutical acceptable salts thereof, comprises a concentration of at least about 0.1% by weight, preferably from about 0.1% to about 10%, more preferably from about 0.5% to about 6%, even more preferably from about 0.5% to about 4%, still more preferably from about 1% to about 4%, most preferably from about 2% to about 4%, per weight and the active ingredient is suspended in a pharmaceutically acceptable carrier. Where the active ingredient is VPA, an acceptable pharmaceutical salt of VPA, an ester, prodrug, or the like derived from VPA, the amount administered is calculated on the basis the parent compound and so the actual weight to be used is increased proportionately.

### Topical application:

In a preferred embodiment of this invention, VPA is used topically applied once to three times daily at a dose of 0.5 mg (milligram) to 200 mg (milligram) per application per lesion of approximately 1 cm² (qcm). In a more preferred embodiment, VPA is used topically applied once to three times daily at a dose of 1.0 mg (milligram) to 100 mg (milligram) per application per lesion of approximately 1 cm² (qcm), and in an even more preferred embodiment, VPA is used topically applied once to three times daily at a dose of 2.0 mg (milligram) to 50 mg (milligram) per application per lesion of approximately 1 cm² (qcm).

The actual daily dose depends on the size of the lesion treated, and accordingly increases with increased lesion size. Multiple lesions of this kind may be treated.

### Pharmakokinetics:

In a preferred embodiment of this invention, VPA is used topically applied once to three times daily. Serum levels of VPA in patients after repeated dose topical application may reach approx. up to 12.0 µg/ml (micogram/milliliter) of VPA when used for the treatment of facial acne vulgaris with a total daily dose of up to 2.000 mg of a 3% VPA containing medication. In a more preferred embodiment, the serum level of VPA is below 8 µg/ml (micogram/milliliter), in a more preferred embodiment, the serum level of VPA is below 6 µg/ml (micogram/milliliter), and in an even more preferred embodiment, the serum level of VPA is below 4 µg/ml (micogram/milliliter). These serum levels may be reached at a daily topically applied total dose of a 3% VPA containing medication of approx. up to 2.000 mg (milligram) which would contain up to 60 mg of VPA.

Topical administration in vivo can be effected in one dose, continuously or intermittently throughout the course of treatment. Methods of determining the most effective means and dosages of administration are well known to those of skill in the art and will vary with the formulation used for therapy, the purpose of the therapy, the target cell being treated, and the subject being treated. Single or multiple administrations can be carried out with the dose level and pattern being selected by the treating physician. In general, a suitable dose of the active compound, i.e., VPA or a VPA derivative, comprises a concentration of at least about 0.1% by weight, preferably 0.1% to 6% per weight, more preferably 0.3% to 5%, more preferably 0.5% to 4% and even more preferably 1% to 4% per weight and the active ingredient is suspended in a pharmaceutically acceptable carrier. Where the active ingredient is a salt, an ester, prodrug, or the like, the amount administered is calculated on the basis the parent compound and so the actual weight to be used is increased proportionately.

If the active compound, i.e., VPA or a VPA derivative, is used in combination with retinoids, a suitable dose of this active compound is in the range of at least 0.1% by weight, preferably 0.1% to 6% per weight, more preferably 0.3% to 5%, more preferably 0.5% to 4% and even more preferably 1% to 4% per weight and for the retinoid used a suitable dose of the active compound is in the range of about 0.01% to about 1% by weight, preferably 0.05% to 0.5% per weight.

In a preferred embodiment of this invention, VPA is used topically applied once to three times daily at an accumulated total daily dose of 0.5 mg (milligram) to 10 mg (milligram) per lesion of approximately 1 cm² (qcm). In a more preferred embodiment, VPA is used topically applied once to three times daily at an accumulated total daily dose of 1 mg (milligram) to 8 mg (milligram) per lesion of approximately 1 cm² (qcm). In an even more preferred embodiment, VPA is used topically applied once to three times daily at an accumulated total daily dose of 2 mg (milligram) to 6 mg (milligram) per lesion of approximately 1 cm² (qcm).

The actual daily dose depends on the size of the lesion treated, and accordingly increases with increased lesion size, including lesion sizes of up to approximately 100 cm² (qcm) with according increases of daily doses.

In other preferred embodiments of the invention the topical formulation containing VPA is made up by one of the three following formulations containing the listed ingredients (content is presented as % [w/w]):

**Formulation example 1:**

| | |
|---|---|
| White mineral oil | 20 |
| Cetyl Alcohol | 24 |
| Cetomacrogoll000 | 6 |
| Valproic Acid | >0.1, e.g., 0.1 to 6 |

| | |
|---|---|
| White vaseline add to 100 | |

**Formulation example 2:**

| | |
|---|---|
| Zinc Oxide | 25 |
| Starch | 25 |
| Valproic Acid | >0.1, e.g., 0.1 to 6 |

| | |
|---|---|
| White vaseline add to 100 | |

**Formulation example 3:**

| | |
|---|---|
| White vaseline | 25 |
| Cetyl Alcohol | 10 |
| Tween 60 | 5 |
| Glycerin | 10 |
| EDTA | 0.2 |

| | |
|---|---|
| Perfume (optionally) 2 drops Sodium Valproate >0.1, e.g., 0.1 to 6 Hidistilled Water add to 10 | |

The various embodiments described herein can be combined with each other.

### Figures

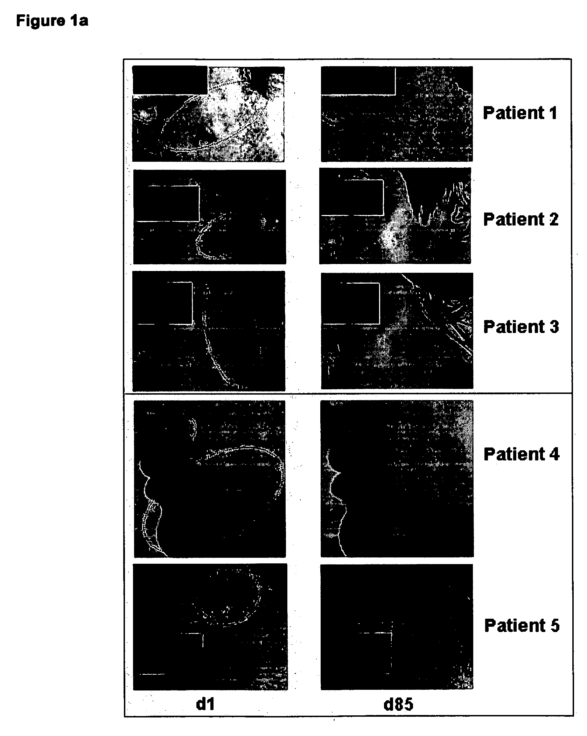

### Figure 1a: Photo documentation of acne vulgaris patients before and after treatment with topically applied VPA

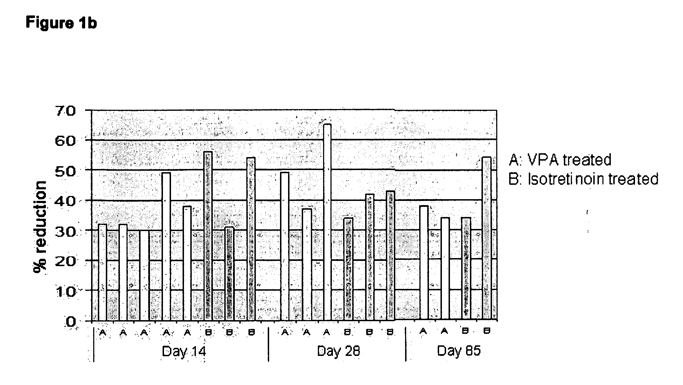

Figure 1 a shows representative photos taken from patients with acne vulgaris before and after 12 weeks of topical treatment with VPA (Patient 1, 2 and 3) or after topical treatment with the retinoid comparator drug isotretinoin (Patients 3 and 4). The lesions seen in these patients before start of treatment (d1; day 1) are displayed on the left (see circles indicating lesion area). These lesions are therapeutically successfully treated as can be seen after the end of treatment on the right (d85; day 85).

### Figure 1b: Time to reduction of the total counts by ≥ 30% of all acne lesions

Figure 1b shows that of the patients that have been treated topically with VPA (A), five patients on day 14, three patients on day 28, and two patients on day 85 displayed for the first time a reduction of ≥ 30% in the total counts of all acne lesions treated. Of those patients being treated with the comparator compound isotretinoin, (B), three patients on day 14, three patients on day 28, and two patients on day 85 displayed for the first time a reduction of ≥ 30% in the total counts of all acne lesions treated.

Based on these findings it surprisingly appears that the topical treatment with VPA has a beneficial therapeutic benefit for the treatment of acne vulgaris which generally is at least comparable to the therapeutic effects obtained with isotretinoin, but may in addition generate a clinical response in an accelerated and therefore faster way.

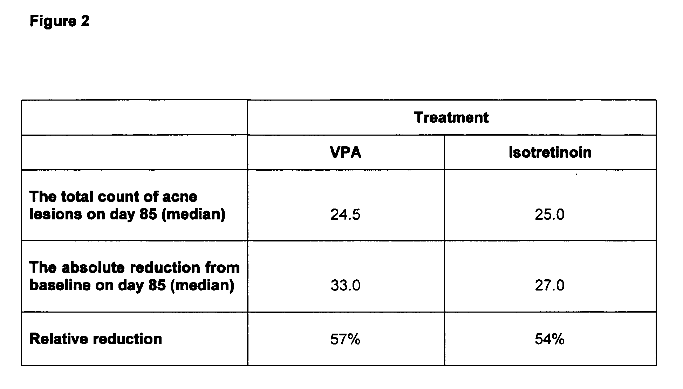

### Figure 2: Clinical therapeutic efficacy data of topical treatment with VPA

Figure 2 shows a summary table of the topical treatment with VPA of patients with acne vulgaris compared to topical treatment with a standard medication comprising the active compound isotretinoin. The table summarizes the reduction of the number of acne lesions as mean values and as percentage relative reduction. Herein, the number of acne lesions at baseline before the start of treatment is compared to the number of acne lesions after treatment (at day 85) for patients treated with VPA and patients treated with isotretinoin. In summary, topical treatment with VPA used as a single active compound surprisingly already resulted in a mean reduction of the number of acne lesions of approx. 57%, whereas the treatment with the comparator isotretinoin resulted in a mean reduction of acne lesions of approx. 54%. These data suggest that the treatment with VPA when used as a mono therapy has a surprisingly comparable therapeutic benefit as the compound isotretinoin which is a marketed standard medication for this indication.

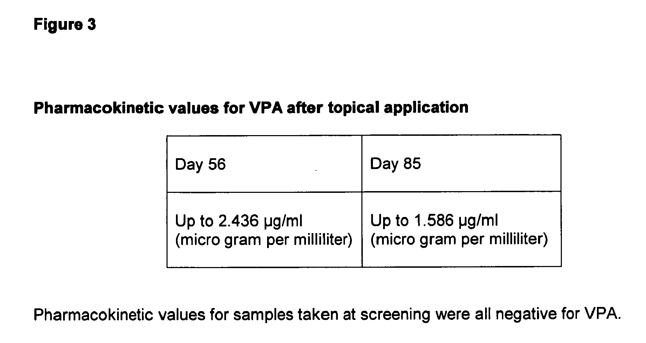

### Figure 3: Pharmakokinetics: Values of VPA content after topical application in patients

Figure 3 represents the serum concentration levels measured in patients treated daily with up to approx. 2.000 mg (milligram) of a 3% VPA containing medication topically applied which refers to approx. 60 mg (milligram) of VPA. Serum level measured reached up to 2.436 µg/ml (micogram/milliliter) of VPA on day 56 and up to 1.586 µg/ml (micogram/milliliter) of VPA on day 85.

### Figure 4: Clinical local tolerability of the topical treatment with VPA

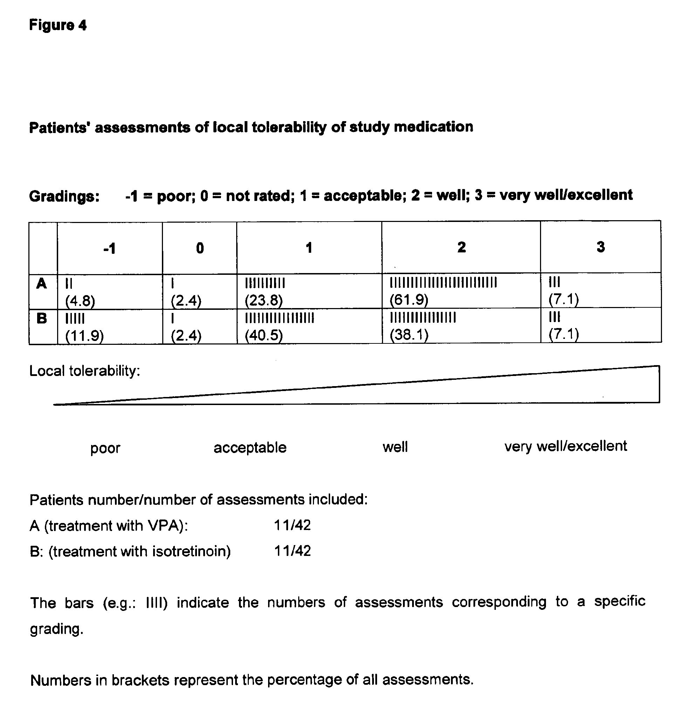

Figure 4 displays a summary table of patients' assessments of local tolerability as a measure of patients compliance and safety in comparison to the local tolerability upon the topical use of isotretinoin. These assessments were scheduled to take place on each visit during the study period. In 61.9% of all assessments of patients that were treated topically with VPA, the local tolerability of this VPA treatment was graded as "well", and in further 23.8% it was graded as "acceptable". In contrast, of the assessments of patients of local tolerability of topical therapy with the retinoid isotretinoin only 38.1 % were graded as "well", and further 40.5% of the assessments of these patients were graded as "acceptable". This shift towards a better local tolerability for the topical therapy with VPA compared to the local tolerability of isotretinoin is also evident in the percentages of patient assessments which recorded only a "poor" tolerability, i.e., only 4.8% of assessments of patients treated with VPA were graded as "poor", whereas 11.9% of assessments of patients treated with isotretinoin were graded as "poor". Note: The bars in figure 4 (such as: IIII) indicate the numbers of assessments corresponding to a specific grading and the numbers in brackets represent the percentage of all assessments.

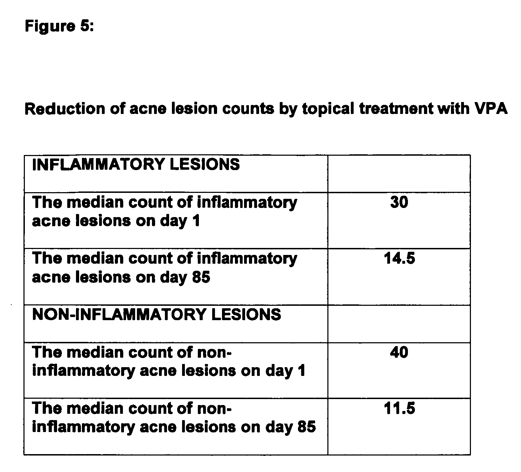

### Figure 5: Reduction of acne lesion counts by topical treatment with VPA

Figure 5 shows that topical treatment with VPA leads to a reduction of both inflammatory and non-inflammatory acne lesions.

### Examples

### Example 1

This invention is based on surprising therapeutic results obtained in a clinical trial performed with patients suffering from mild to moderate acne vulgaris. This study was carried out as a double-blind, randomized study. Under informed consent patients with mild to moderate facial acne vulgaris were enrolled in the study. The treatment period was 12 weeks.

### Method:

### Diagnosis and Main Criteria for Inclusion

Patients aged between 18 and 35 years with acne vulgaris of the facial skin requiring a topical treatment were selected for the study with the main inclusion criteria being confirmed diagnosis of facial acne comedonica or acne papulopustulosa with a mild to moderate intensity (grade 2 to 8 according to the revised Leeds Scale of Acne Grading).

### Test Product, Dose and Mode of Administration

VPA was provided as a clear off-white, semi-solid 3% gel (30 mg/ml VPA). After washing the face with a mild cleanser, the gel was applied daily to the clean and dry skin once daily.

### Reference Therapy and Placebo, Dose and Mode of Administration

As a comparator reference therapy the retinoid isotretinoin was provided as a 0.05% gel (0.5 mg isotretinoin/g).

### Criteria for Evaluation

### Efficacy

- Assessment of the therapeutic efficacy compared to the baseline was evaluated separately by the investigator and by the patient according to a 7-point scale
- Counting of lesions in the entire face
- Investigator's overall assessment of improvement by means of a photo documentation.

### Safety and Tolerability

- Safety laboratory parameters
- Clinical safety examinations
- Adverse events
- Patient's assessment of local tolerability according to a 5-point scale.

### Study endpoints:

**Primary endpoint:**

| | | |
|---|---|---|
| efficacy: | - | change of total count of facial acne lesions from baseline to the end of week 12 (comparing day 1 to day 85, i.e., d1, d85) |
| Secondary endpoints efficacy: | - | time to reduction of acne lesions by > 30% |
| | - | count of acne lesions in the face after 2, 4, 8 and 12 weeks of treatment and its percentage and absolute change to baseline |
| safety/tolerability: | - | safety laboratory parameters |
| | - | clinical safety |
| | - | patient's assessment of local tolerability |

In the clinical study described in Example 1, also the time to reduction of the total counts by ≥ 30% of all acne lesions treated was investigated on each visit of the patients to the study hospital. Once the reduction had reached ≥ 30%, the actual at this point estimated reduction rate and the day of the visit was recorded. The data obtained are shown in figure 1b, representing the actual observed reduction rates once they have reached ≥ 30% on the corresponding hospital visits on day 14, day 28 or day 85.

### Results:

In Figure 1 a representative pictures of patients treated topically with VPA alone (Patient 1, 2 and 3) or of patients treated topically with the retinoid comparator isotretinoin (Patients 4 and 5) are presented. The lesions seen in all patients before start of treatment on the left (d1) (see circles indicating lesion area) are therapeutically successfully treated as can be seen after the end of treatment on the right (d85).
Furthermore, the time to reduction of the total counts by ≥ 30% of all acne lesions treated are shown in figure 1b, representing the actual observed reduction rates once they have reached ≥ 30% on the corresponding patients' hospital visits on day 14, day 28 or day 85. Surprisingly, treatment with VPA resulted in an accelerated onset of clinical responses when compared to the therapy with isotretinoin. At day 14, there were 5 patients that were treated with VPA reported to already display a reduction of their total facial acne lesion counts by at least 30%, whereas only 3 patients treated with isotretinoin displayed such a reduction of their total facial acne lesion counts by at least 30%.

### Example 2

Based on the clinical study described in Example 1, a summary table of results was assembled and is shown in figure 2. This table summarizes the percentage change for the acne lesion counts in the face of the patients treated, comparing the lesion counts on day 1 (before therapy start) and after the end of therapy on day 85 (d85).

### Results:

The table of figure 2 summarizes the reduction of the number of acne lesions as mean values and as percentage relative reduction. Herein, the number of acne lesions at baseline before the start of treatment is compared to the number of acne lesions after treatment (at day 85). In summary, when compared to baseline lesion counts, at day 85 topical treatment with VPA used as the only active treatment surprisingly resulted in a mean reduction of the number of acne lesions of approx. 57%, whereas the treatment with the comparator isotretinoin resulted in a mean reduction of acne lesions of approx. 54%. These data suggest that the treatment with VPA when used as a mono therapy is surprisingly of comparable therapeutic benefit as the compound isotretinoin which is marketed for this medical indication.

### Example 3

In parallel, an analysis of the VPA content in the blood of acne vulgaris patients treated topically with a 3% VPA containing medication was performed using HPLC based methodology, and the results are presented in figure 3. Serum level measured reached up to 2.436 µg/ml of VPA on day 56 and up to 1.586 µg/ml of VPA on day 85. These serum concentration levels were measured after daily treatment with up to approx. 2.000 mg of a 3% VPA containing medication topically applied which refers to a total daily dose of VPA approx. 60 mg. If more or larger lesions would be treated, and therefore higher daily amounts of VPA would be topically applied, it is expected that accordingly higher serum levels may be obtained.

In addition to the assessment of the therapeutical efficacy, the patients' assessments of local tolerability and the side effect profile was recorded in this clinical study described in example 1. Figure 4 summarizes the patients' assessments of local tolerability which was recorded on each visit of the patients during the study period which took place on days 14, 24, 56 and 85. The patients assessed their perception of local tolerability according to the following grading scheme:

| **-1** | **0** | **1** | **2** | **3** |
|---|---|---|---|---|
| poor | not rated | acceptable | well | Very well/ excellent |

### Results:

Data of the patients' assessment of local tolerability of the studied medications is summarized in figure 4. In 61.9% of all assessments of patients which were treated topically with 3% VPA, the local tolerability of this VPA treatment was graded "well", and in further 23.8% it was graded as "acceptable". In contrast, of the assessments of patients of local tolerability of topical therapy with the retinoid isotretinoin, only 38.1 % were graded as "well", and further 40.5% of the assessments of these patients were graded as "acceptable". This shift towards a better local tolerability for the topical therapy with VPA compared to the local tolerability of isotretinoin is also evident in the percentages of patients' assessments which recorded only a "poor" tolerability, i.e., only 4.8% of assessments of patients treated with VPA were graded as "poor", whereas 11.9% of assessments of patients treated with isotretinoin were graded as "poor".

Adverse events observed in patients treated topically with VPA consisted primarily of mild to moderate administration site conditions, such as site irritation, site erythema or site pain. All adverse events were resolved after the end of the clinical study.

### Example 4

In the clinical study described in Example 1, the counts of acne lesions treated were also assessed as counts of different types of lesions separately, i.e., recording the number of inflammatory lesions, and recording the number of non-inflammatory lesions separately.

### Results:

Figure 5 displays the reduction of inflammatory and non-inflammatory lesion counts of acne patients treated topically with VPA. The median counts of inflammatory lesions on day 1 before start of treatment was estimated to be 30, and at the end of treatment on day 85 was assessed to be reduced to 14.4 lesions. In parallel, the median counts of non-inflammatory lesions on day 1 before start of treatment was estimated to be 41, and at the end of treatment on day 85 was assessed to be reduced to 11.5 lesions.

These data show that VPA surprisingly does not only reduce the number of total acne lesion counts, but furthermore unexpectedly reduces at the same time the counts of inflammatory and non-inflammatory lesions to a similar extent.

## Claims

1. The use of a histone deacetylase inhibitor for the manufacture of a medicament for the topical treatment of mild or moderate acne vulgaris.

2. The use according to claim 1, wherein the medicament is to be used for the treatment of non-inflammatory acne lesions.

3. The use according to claim 1 or 2, wherein said histone deacetylase inhibitor is selected from the group consisting of valproic acid (VPA) and pharmaceutically acceptable derivatives and prodrugs of VPA.

4. The use according to claim 1 or 2, wherein said histone deacetylase inhibitor is selected from the group consisting of hydroxamic acid derivatives, benzamides, pyroxamides and derivatives thereof, microbial metabolites exhibiting HDAC inhibitory activity, fatty acids and derivatives thereof, cyclic tetrapeptides, peptidic compounds, HDAC class III inhibitors and SIRT inhibitors.

5. The use according to claim 4, wherein said histone deacetylase inhibitor is selected from the group consisting of LAQ824, LBH589, Trichostatin A, Suberoyl anilide hydroxamic acid, CBHA, Pyroxamide, Scriptaid, CI-994, CG-1521, Chlamydocin, Biaryl hydroxamate such as A-161906, Bicyclic aryl-N-hydroxycarboxamides, PXD-101, MGCD0103, TPX-HA analogue (CHAP), Oxamflatin, Trapoxin, Depudecin, Apidicin, MS-275, Pivanex (Pivaloyloxymethyl butyrate), trapoxin A, Depsipeptide (FK-228), Tacedinaline, MG2856.

6. The use according to any one of the preceding claims, wherein said composition comprises said histone deacetylase inhibitor at a concentration of from 1% to 4% by weight, and the active ingredient is suspended in a dermatologically acceptable carrier.

7. The use according to any one of the preceding claims, wherein said histone deacetylase inhibitor is topically applied once to three times daily at an accumulated total daily dose of from 0.5 mg to 200 mg per 1 cm² of lesion.

8. The use according to claim 7, wherein said accumulated total daily dose is from 2.0 mg to 50 mg per 1 cm² of lesion.

9. The use according to any one of the preceding claims, wherein the total amount of topically applied medicament is 2.000 mg or less per day, and the total amount of the topically applied histone deacetylase inhibitor is 60 mg or less per day.

10. The use according to any one of the preceding claims, wherein the serum concentration of said histone deacetylase inhibitor in a treated patient is less than 12.0 µg/ml after continued treatment over a period of at least 56 days.

11. The use according to claim 10, wherein said serum concentration is below 4 µg/ml.

12. The use according to any one of the preceding claims, wherein the treatment does not cause erythema or pain at the site of application.

13. The use according to any one of the preceding claims, wherein the local tolerability of the medicament is higher than that of isotretinoin.

14. The use according to any one of the preceding claims, wherein said treatment further comprises administration of a further active agent.

15. The use according to claim 13, wherein said further active agent is not a histone deacetylase inhibitor.

16. The use according to claim 15, wherein said further active agent is selected from the group consisting of antibiotics, retinoids, hormonal agents and topical bactericidals.

17. The use according to any one of claims 1 to 13, wherein said medicament comprises one histone deacetylase inhibitor as the only active agent for treating acne.

18. A topical pharmaceutical composition comprising (i) a first active agent which is a histone deacetylase inhibitor, and (ii) a second active agent which is selected from the group consisting of histone deacetylase inhibitors other than the first active agent, antibiotics, retinoids, hormonal agents and topical bactericidals.

19. A pharmaceutical kit for the treatment of acne, comprising (i) a first active agent which is a histone deacetylase inhibitor, and (ii) a second active agent which is selected from the group consisting of histone deacetylase inhibitors other than the first active agent, antibiotics, retinoids, hormonal agents and topical bactericidals.
